# EUROPEAN PATENT SPECIFICATION

(11) **EP 0 468 457 B1**
(45) Date of publication and mention of the grant of the patent: **06.03.1996**
(21) Application number: 91112343.8
(22) Date of filing: 23.07.1991
(51) Int. Cl.: C07D 211/22, A61K 31/445, A01N 43/40

(54) **Novel substituted piperidines and their use as inhibitors of cholesterol synthesis**
Neue substituierte Piperidine und ihre Verwendung als Inhibitoren der Cholesterol-Synthese
Nouvelles pipéridines substituées et leur application comme inhibiteurs de la synthèse du cholestérol

(30) Priority: 24.07.1990 US 557855
(43) Date of publication of application: 29.01.1992
(73) Proprietor: MERRELL DOW PHARMACEUTICALS INC., Cincinnati Ohio 45215 (US)
(72) Inventor: McCarthy, James R., West Chester, Ohio 45069 (US); Barney, Charlotte L., Cincinnati Ohio 451212 (US); Wannamaker, Marion W., West Chester, Ohio 45069 (US)
(74) Representative: VOSSIUS & PARTNER

(56) References cited:
- DE-A- 3 433 036
- US-A- 3 178 439
- CHEMICAL ABSTRACTS, vol. 79, No. 23, December 10, 1973, Columbus, Ohio, USA FERLES et al. "Synthesis of some 1-methyl- -4-(hydoxyalkyl)piperdines" page 364, column 1, abstract-no. 136 948d

## Description

The present invention relates to a group of compounds which are novel substituted piperidines and which act to inhibit the synthesis of cholesterol in mammals and in fungi.

Vascular disease, because of its effects upon the brain, heart, kidneys, extremities, and other vital organs, is a leading cause of morbidity and mortality in the United States and in most Western countries. In this regard, much has been learned about arteriosclerosis, atherosclerosis, and the lipidemias, with particular reference to cholesterol. In particular, there is convincing evidence of a reciprocal relationship between a high serum cholesterol and the incidence of atherosclerosis and its complications. Much interest has been expressed in recent years in reducing the level of serum cholesterol. However, some studies have shown that even radical reductions in dietary cholesterol achieves only a modest decrease of 10 to 15% in plasma cholesterol. Thus, it has been appreciated that further reductions in serum cholesterol will require other therapeutic measures, including the physiological inhibition of cholesterol synthesis in the body.

The enzymatic biosynthesis of cholesterol is a complex process, which requires altogether some 25 reaction steps. The pathway can be divided into three stages: (1) the conversion of acetic acid to mevalonic acid; (2) the conversion of mevalonic acid into squalene; and (3) the conversion of squalene into cholesterol. In the last stage of cholesterol biosynthesis, squalene is converted to squalene 2,3-epoxide via oxidation, a reaction catalyzed by squalene monooxygenase, also known as squalene epoxidase. The squalene 2,3-epoxide then undergoes cyclization to lanosterol, the first sterol to be formed.

The cyclization of 2,3-oxidosqualene to lanosterol is a key reaction in the biosynthesis of cholesterol in animals. The reaction is catalyzed by the microsomal enzyme 2,3-oxidosqualene lanosterol-cyclase. (See generally, Taylor, Frederick R., Kandutsch, Andrew A., Gayen, Apurba K., Nelson, James A., Nelson, Sharon S., Phirwa, Seloka, and Spencer, Thomas A., *24,25-Epoxysterol Metabolism in Cultured Mammalian Cells and Repression of 3-Hydroxy-3-methylglutaryl-CoA Reductase,* The Journal of Biological Chemistry, 261, 15039-15044 (1986), incorporated herein by reference.)

In addition, it has recently been reported that certain compounds, such as allylamines, act as potent inhibitors of fungal squalene epoxidase. Fungal infections (mycoses) are found throughout the world. Only a few structural classes of compounds currently satisfy the demands of modern chemotherapy in their treatment and the search for new types of active substances is of major therapeutic importance. (See generally, Stutz, Anton, *Allylamine Derivatives-A New Class of Active Substances in Antifungal Chemotherapy,* Angew. Chem. Int. Ed. Engl., 26 (1987) 320-328.) As inhibitors of squalene epoxidase in animals, the compounds of the present invention are believed to be useful in the treatment of fungal infections through the inhibition of cholesterol synthesis.

The present invention relates to compounds having the following general formula: 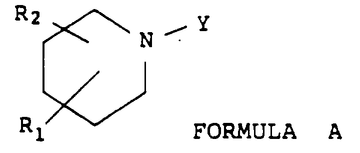 and the pharmaceutically acceptable salts thereof wherein Y is -A-(Alk₁)-D-(Alk₂)-E-(Alk₃)-CH₃ wherein, A is -CH₂-, 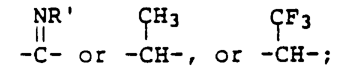 D and E are each independently -CH2-, 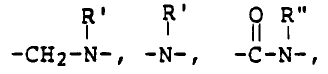 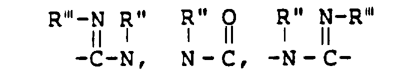 or a direct bond, with the proviso that when D is a moiety from the group 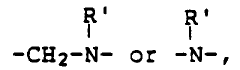 E cannot be a moiety from the same group, and that when D is a moiety form the group 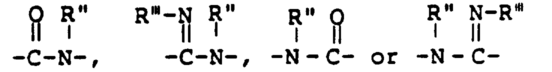 E cannot be a moiety from the same group; and, with the proviso that when A is -CH(CF₃), D and/or E cannot be a moiety from the group 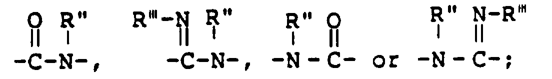
(Alk₁), (Alk₂) and (Alk₃) are each independently a straight chain alkylene moiety containing from 0 to 5 carbon atoms, optionally substituted with from 1 up to 3 methyl groups, with the proviso that Alk₂ cannot have the value of 0 carbon atoms; or,
(Alk₁), (Alk₂) and (Alk₃) are each independently a straight chain alkenylene moiety containing from 2 up to 6 carbon atoms, the straight chain alkenylene moiety having 1 to 2 double bonds, and optionally substituted with from 1 up to 3 methyl groups;

- R₁: is a group of the formula 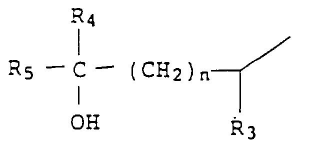 positioned in either the 3- or the 4-position of the piperidine ring, wherein R₃ is (C₁-C₄)lower alkyl, R4 and R₅ are each independently hydrogen or (C₁-C₄)lower alkyl and n is zero or an integer from 1 to 3;
- R₂: is hydrogen, hydroxy or (C₁-C₄) lower alkyl; and
- R', and R'' and R''': are each independently hydrogen or (C₁-C₄)-lower alkyl.

A preferred embodiment of the invention is a compound in which R₁ is a group of the formula:

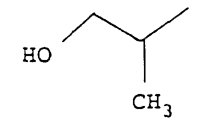

As used in this application:
(a) the term alkylene refers to methylene, ethylene, propylene, butylene, pentylene and hexylene;
(b) the term alkenylene refers to any of the above alkylenes having 1 or 2 double bonds along the chain thereof;
(c) the term (C₁-C₃)lower alkyl refers to methyl, ethyl, propyl and isopropyl;
(d) the term (C₁-C₄) lower alkyl refers to methyl, ethyl, propyl, isopropyl, butyl, sec-butyl and tert-butyl.

Also, as used in this application, the substituent represented as R₂ may be at any position 2-6 around the piperidine ring, except at the position occupied by R₁. There may be up to three such independent substitutions around the piperidine ring wherein the substituent is other than hydrogen.

In general, compounds of the present invention are prepared by the following methods. Synthesis of all the final products of this invention begin with the synthesis of the intermediate A as described in detail below.

### PREPARATION OF 3- AND 4-SUBSTITUTED PIPERIDINE INTERMEDIATES

Intermediate A, shown below in Reaction Scheme A wherein R₁ and R₂ are defined as above, can be prepared as follows when R₄ and R₅ are hydrogen.

### REACTION SCHEME A

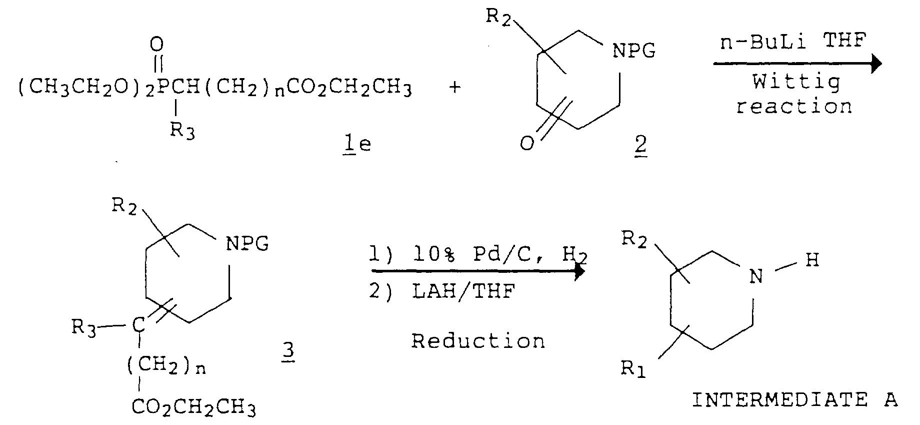

First, a Wittig type reaction is performed in which an appropriate alkyl phosphonate 1e wherein n is zero or an integer from 1 to 3 and R₃ is (C₁-C₄)lower alkyl, is reacted with a 3- or 4-piperidinone 2 to form intermediate substituted piperidine 3. The compounds 1e and 2 are either commercially available or are readily prepared by techniques well-known in the art.

The piperidinone 2 has a protecting group (PG) at the nitrogen position. Appropriate protecting groups are well-known in the art and include benzyl, benzyloxy, p-methoxybenzyl, as well as other protecting groups, which should not be construed as limiting. The alkyl phosphonate 1e is chosen such that R₃ and n have the same definitions as that desired in the final product. If will be understood that while the alkyl phosphonate represented by structure 1e is a triethyl ester, other alkyl esters such as the methyl, propyl or isopropyl esters, for example, may also be utilized and this should not be construed as limiting.

The alkyl phosphonate 1e is dissolved in an anhydrous, aprotic solvent such as tetrahydrofuran (THF) to which a solution of n-butyllithium in hexane, or a similar basic agent, is added in an approximately equimolar amount at low temperature (-78°C). The appropriate piperidinone 2 in THF is added dropwise to the reaction mixture in approximately an equimolar ratio to the alkyl phosphonate 1e The piperidinone 2 is chosen such that the location of the carbonyl oxygen is the same as the location of the substitution desired in the final product, i.e. either in the 3- or the 4-position. The reaction is warmed to room temperature and is performed preferably under an inert atmosphere such as argon. The intermediate 3 can be separated from solution and purified by techniques well-known in the art. For example, the reaction can be diluted with a saturated solution of ammonium chloride, washed with a 10% sodium hydroxide solution, dried over magnesium sulfate and evaporated to give a crude oil. The crude oil can be purified by techniques well-known in the art such as chromatography.

The purified intermediate substituted piperidine 3 is then reduced to Intermediate A wherein R₁ is defined as above, in two steps as follows. This reduction achieves three results. The carbon double bonded to the piperidine is hydrogenated; the terminal ester is reduced to -CH₂OH; and the protecting group is cleaved from the nitrogen of the piperidine ring. The intermediate 3 is first converted to the corresponding acid addition salt by treatment with a saturated solution of hydrochloric acid in methanol, for example, followed by removal of the solvent. The reduction is performed by techniques well-known in the art. For example, the residue is dissolved in dry ethanol and transferred to a Parr hydrogenation flask. A catalytic amount of 10% palladium (Pd/C) is added and the vessel charged with hydrogen to 344.6 KPa (50 psi). After shaking for several hours, the catalyst is filtered, through Celite for example, and the solvent removed *in vacuo.* After removal of the solvent, the residue is neutralized with 10% sodium hydroxide solution.

The resulting product is extracted by techniques well-known in the art. For instance, the aqueous layer is extracted with ether and the extracts are dried over magnesium sulfate. Removal of the solvent *in vacuo* gives an oil, which is then dissolved in dry THF and treated with excess lithium aluminum hydride (LAH). The reaction is quenched by techniques well-known in the art, such as described in Fieser & Fieser, Vol. 1, page 584, and the solvent removed to give an oil that is purified by techniques known in the art. For example, the oil can be purified using chromatography on silica gel using a 25% ethyl acetate:hexane solution followed by a 20% methanol:chloroform solution as the eluents. Concentration of the methanol:chloroform fractions gives the desired product, Intermediate A.

### REACTION SCHEME I

Compounds according to Formula I can be made according to the following reaction scheme. 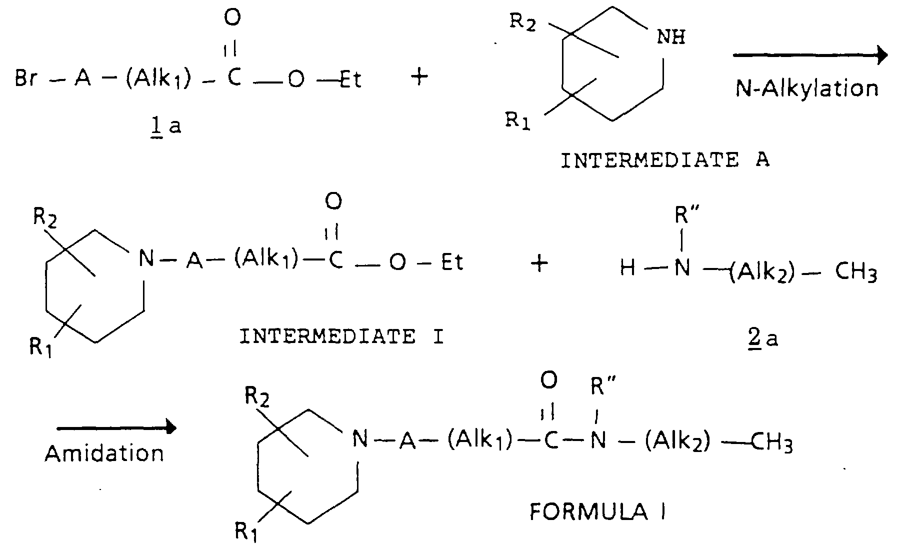 Formula I is a representation of Formula A wherein D is 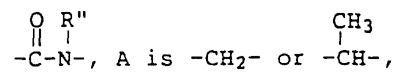 (Alk₃) is an alkylene moiety of 0 carbon atoms, (Alk₁) and (Alk₂) and R" are defined as in Formula A, and E is a direct bond.

The first step in the reaction is the N-alkylation of the piperidine Intermediate A by a bromoalkyl ester compound represented by structure 1a It will be understood that the abbreviation Br in this example and as used throughout this application represents bromine; however, the chloroalkyl ester compound may also be utilized. Esters other than ethyl (Et) esters, such as methyl, n-propyl, or isopropyl, may also be used. Intermediaire A is substituted by R₁ and R₂ as defined above.

The appropriate starting compounds are a bromoalkyl ester 1a wherein A and (Alk₁) have the same definitions as that desired in the final product and the Intermediate A wherein R₁ and R₂ have the same definition as that desired in the final product, as represented by Formula I. The compound 1a is either commercially available or is readily prepared by techniques well-known in the art.

The alkylation reaction can be performed by techniques well known in the art. Typically the bromoalkyl ester 1a and the Intermediate A are mixed in approximately a 1:2 molar ratio in a solvent, such as benzene, and the reaction mixture is heated at reflux under an inert atmosphere for approximately 16 hours. Alternatively, the bromoalkyl ester 1a Intermediate A and triethylamine may be mixed in equimolar amounts. Intermediate I is then recovered from the reaction mixture and purified by techniques known in the art. For example, the reaction mixture is concentrated under reduced pressure and then taken up in ether and filtered to give Intermediate I as shown, which can be purified by flash chromatography.

The next step in the reaction scheme is an amidation reaction between Intermediate I and a substituted amine as shown in structure 2a The compound 2a is either commercially available or is readily prepared by techniques well-known in the art. The substituted amine 2a chosen is one in which R" and (Alk₂) have the same definition as R" and (Alk₂) in the final product, represented by Formula I. The substituted amine 2a and Intermediate I are contacted in a 1:1 to a 2:1 molar ratio in the presence of 2-hydroxypyridine and heated at approximately 60°C for several hours. The solution is poured into water, extracted with ethyl acetate, dried over magnesium sulfate and concentrated under reduced pressure. The final product is further purified using chromatographic techniques well known in the art such as flash chromatography.

### REACTION SCHEME II

An amide according to Formula I, as prepared and defined above, can be reduced to the corresponding amine, as shown below in Formula II as follows: 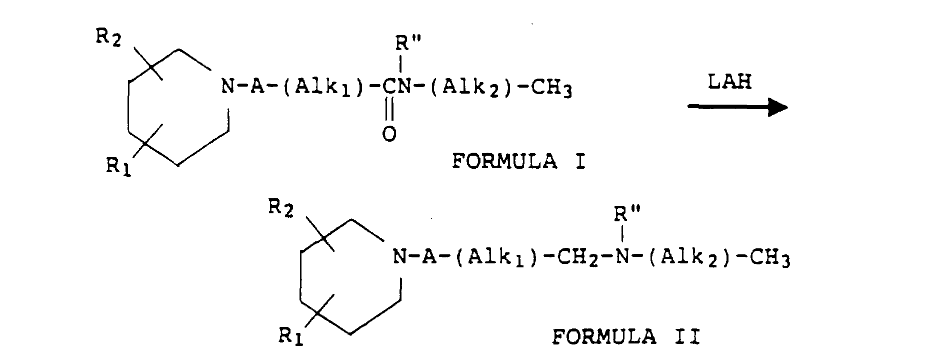 Formula II is a representation of Formula A wherein D is 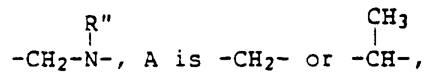 A is -CH₂- or E is a direct bond, (Alk₃) is an alkylene moiety of 0 carbon atoms, and (Alk₁), (Alk₂), R₁, R₂ and R" are defined as in Formula I.

The amide made by the method of Reaction Scheme I is dissolved in THF. The solution is cooled to about 10°C and a reducing agent, such as lithium aluminum hydride (LAH), is added. The reaction is stirred overnight at room temperature for example, followed by heating the reaction at reflux.

The solution is then cooled to room temperature, quenched by techniques well-known in the art, such as described in Fieser & Fieser, *supra,* and the solvent removed. The resulting oil can be purified by techniques well known in the art. The resulting oil can be dissolved in ether, filtered and treated with anhydrous hydrochloric acid. The resulting precipitate is filtered and recrystallized in ethyl acetate/isopropyl alcohol, for example, to give the final product according to Formula II.

### REACTION SCHEME III

Alternatively, the amide according to Formula I, as prepared and defined above, can be converted to the corresponding amidine, according to Formula III. 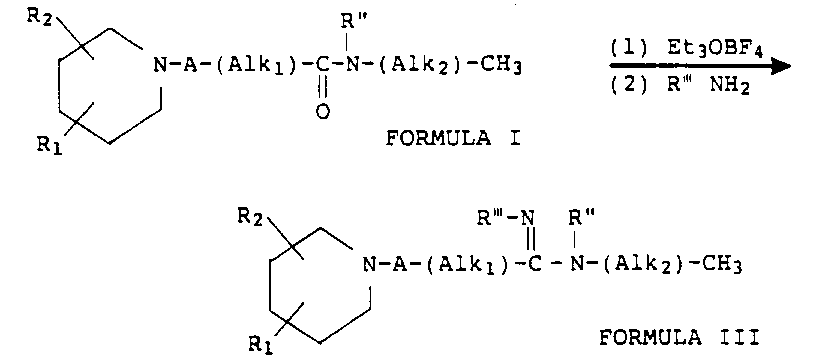 Formula III is a representation of Formula A wherein D is 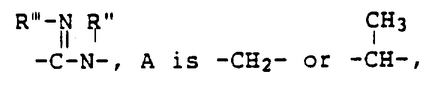 (Alk₃) is an alkylene moiety of 0 carbon atoms, E is a direct bond, and (Alk₁), (Alk₂), R₁, R2, and R" are defined as in Formula A.

The amide of Formula I is allowed to react with triethyloxonium tetrafluoroborate (Et₃OBF₄) followed by addition of an amine of the structure R"'-NH₂. The amine is chosen such that R"' has the same definition as the definition of R"' desired in the final product.

An amide prepared according Reaction Scheme I is dissolved in methylene chloride and treated with approximately an equimolar amount of triethyloxonium tetrafluoroborate followed by an excess amount of the desired substituted amine. The reaction is stirred overnight at room temperature followed by heating the reaction at reflux.

The solution is then cooled to room temperature, quenched with aqueous sodium hydroxide, extracted into organic solvent, dried over a drying agent such as magnesium sulfate and concentrated under reduced pressure. The resulting oil can be purified by techniques well-known in the art. The resulting oil can be dissolved in ether, filtered and treated with anhydrous hydrochloric acid. The resulting precipitate is filtered and recrystallized in ethyl acetate/isopropyl alcohol, for example, to give the final product according to Formula III.

### REACTION SCHEME IV

Amidines according to Formula IV can be prepared by the following reaction scheme when R" is hydrogen. Formula IV is a representation of Formula A wherein D is 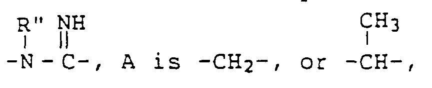 (Alk₃) is an alkylene moiety of 0 carbon atoms, E is a direct bond and (Alk₁), (Alk₂), R₁, R₂ and R" are defined as in Formula A.

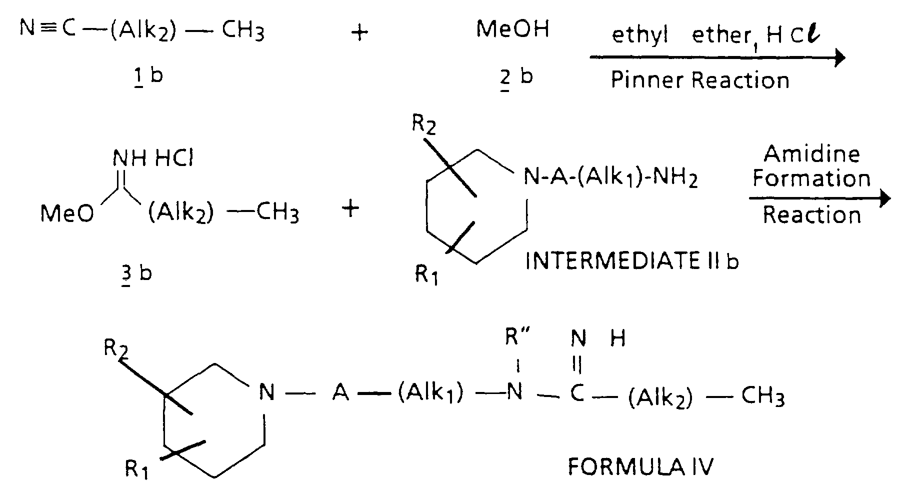

The first step in the reaction sequence is to conduct a Pinner reaction converting alkylnitrile 1b wherein (Alk₂) has the same definition as that desired in the final product, to the imidate ester 3b The compound 1b is either commercially available or is readily prepared by techniques well-known in the art. The alkylnitrile 1b and an appropriate alcohol such as methanol 2b are mixed in approximately equimolar amounts in a solvent, such as ethyl ether and cooled to 0°C, then saturated with hydrochloric acid and stirred overnight at 0°C to room temperature. The imidate ester 3b is concentrated under reduced pressure and can be purified by standard techniques such as trituration in ether, filtration and air drying. The resulting salt is then mixed with an equimolar amount of the appropriate N-substituted alkylamine piperidine represented by Intermediate IIb in a amidine formation reaction.

Intermediate II can be made as follows:

### REACTION SCHEME IVA

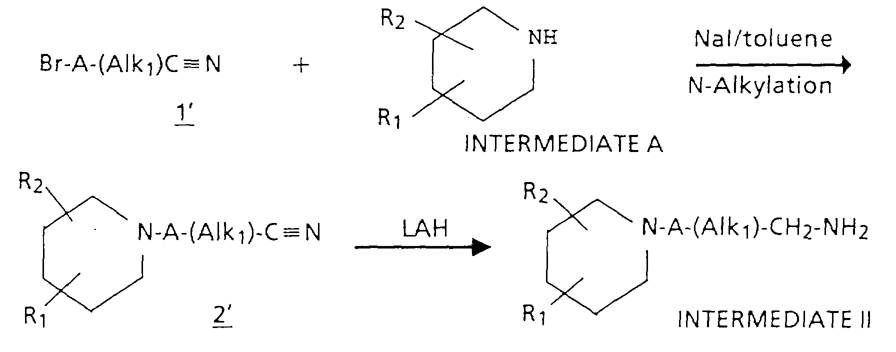

The Intermediate A is chosen such that R₁ and R₂ have the same definition as that desired in the final product and the bromoalkylcyano compound 1' is chosen such that A and (Alk₁) have the same definitions as that desired in the final product. The compound 1' is either commercially available or is readily prepared by techniques well-known in the art. The reactants are combined in an inert solvent such as toluene with a catalytic amount of sodium iodide and heated at reflux. The alkylcyano piperidine product 2' is isolated by partitioning between ether and sodium bicarbonate.

The alkylcyano piperidine 2' is then mixed, dropwise, with an approximately equimolar amount of lithium aluminum hydride powder in tetrahydrofuran. The reaction is stirred at room temperature overnight, quenched with water and sodium hydroxide, filtered, washed, and dried over magnesium sulfate, and concentrated under reduced pressure to give Intermediate II.

Intermediate II and the imidate ester hydrochloride 3b are then mixed in approximately equimolar amounts and allowed to stand overnight at room temperature. The final product according to Formula IV can be purified by standard techniques, such as recrystallization from ethyl acetate/ isopropyl alcohol, for example, to give the final product according to Formula IV.

### REACTION SCHEME V

The above amidine according to Formula IV, as prepared and defined above, can be hydrolyzed by treatment with aqueous sodium hydroxide to form an amide according to Formula V.

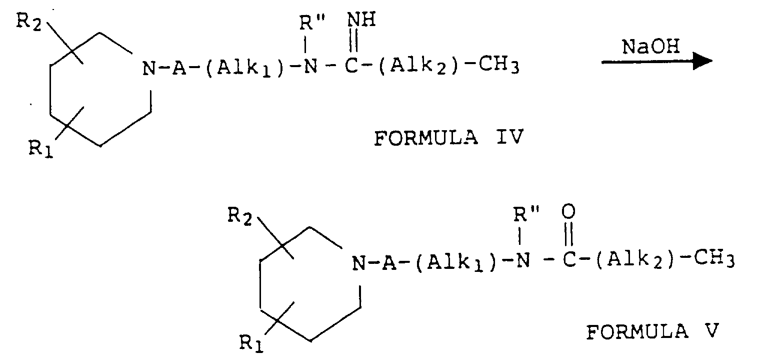

Formula V is a representation of Formula A wherein D is 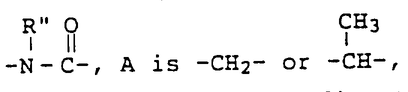 (Alk₃) is an alkylene moiety of 0 carbon atoms, E is a direct bond, and (AlK₁), (AlK₂) R₁, R₂, and R" are defined as in Formula A. The final product of Formula V as its hydrochloric acid salt can be collected by filtration and air dried.

### REACTION SCHEME VI

The amide of Formula V, as prepared and defined above, can be treated with an alkylating agent, such as triethyloxonium tetrafluoroborate, followed by introduction of an excess amount of a substituted amine H₂NR''' to form a substituted amidine according to Formula VI. 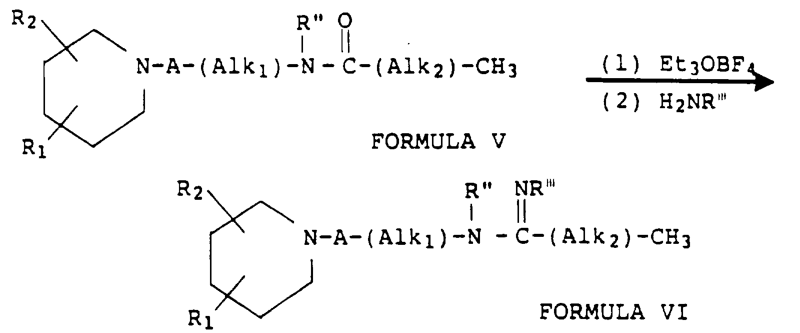 Formula VI is a representation of Formula A wherein D is 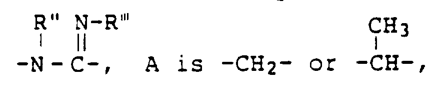 (Alk₃) is an alkylene moiety of 0 carbon atoms, E is a direct bond, and (Alk₁), (Alk₂) R₁, R₂, and R" are defined as in Formula A. The amine H₂NR"' is the chosen such that R''' has the same definition as that desired in the final product. The final product according to Formula VI can be purified according to techniques well-known in the art. The resulting oil can be dissolved in ether, filtered and treated with anhydrous hydrochloric acid. The resulting precipitate is filtered and recrystallized in ethyl acetate/isopropyl alcohol, for example, to give the final product according to Formula VI.

In addition to the above, complex compounds in which the alkyl side chain can include both an amine and an amide, or an amine and an amidine or an amide and an amidine can be prepared by the following reaction schemes.

### REACTION SCHEME VII

Compounds according to Formula VII can be made according to Reaction Scheme VII. Formula VII is a representation of Formula A wherein A is -CH₂- or 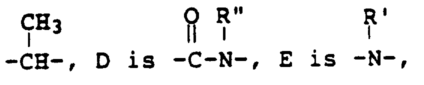 and (Alk₁), (Alk₂), (Alk₃) R" , R' , R₁ and R₂ are defined as above in Formula A.

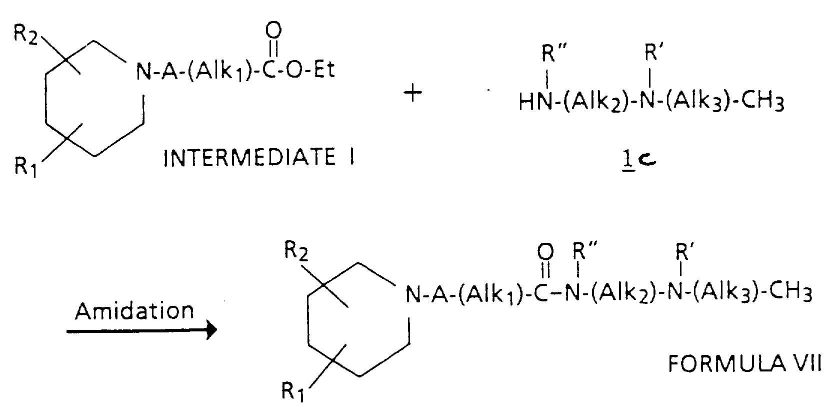

Intermediate I is prepared as described in detail above in Reaction Scheme I. Intermediate I is then contacted with the diamine 1c in approximately a 1:1 to a 1:2 molar ratio in the presence of 2-hydroxypyridine. The diamine compound 1c is either commercially available or is readily prepared by techniques well-known in the art. The diamine compound 1c is chosen such that R', R", (Alk₂) and (Alk₃) have the same definition as that desired in the final product as represented by Formula VII. The reactants are then heated to approximately 60°C.

The resulting compound according to Formula VII can be extracted and purified according to methods known in the art. The resulting oil can be dissolved in ether, filtered and treated with anhydrous hydrochloric acid. The resulting precipitate is filtered and recrystallized in ethyl acetate/isopropyl alcohol, for example, to give the final product according to Formula VII.

### REACTION SCHEME VIII

Further treatment of a compound according to Formula VII, as prepared and defined above, with an alkylating agent, such as triethyloxonium tetrafluoroborate, followed by the introduction of an excess amount of a substituted amine H₂NR''' results in a compound according to Formula VIII. 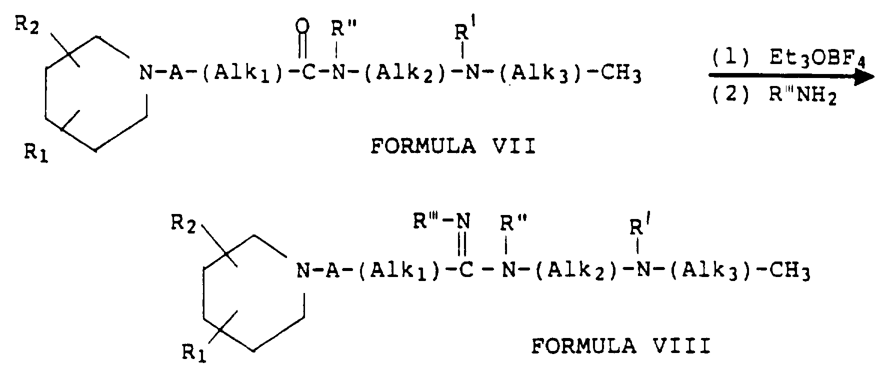 Formula VIII is a representation of Formula A wherein A is -CH₂- or 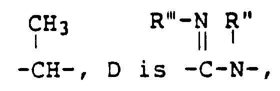 E is 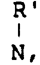 and (Alk₁), (Alk₂)_{,} (Alk₃) R', R", R₁, and R₂ are defined as above in Formula A.

The amine H₂NR''' is chosen so that R" has the same definition as the definition of R''' desired in the final product. An amide prepared according Reaction Scheme VII is dissolved in methylene chloride and treated with approximately an equimolar amount of triethyloxonium tetrafluoroborate followed by an excess amount of the desired substituted amine. The final product according to Formula VIII can be purified by techniques well-known in the art. The reaction is stirred overnight at room temperature followed by heating the reaction at reflux. The solution is then cooled to room temperature, quenched with aqueous sodium hydroxide, extracted into organic solvent, dried over a drying agent such as magnesium sulfate and concentrated under reduced pressure. The resulting oil can be purified by techniques well-known in the art. The oil can then be dissolved in ether, filtered and treated with anhydrous hydrochloric acid. The resulting precipitate is filtered and recrystallized in ethyl acetate/isopropyl alcohol, for example, to give the final product according to Formula VIII.

### REACTION SCHEME IX

Compounds according to Formula IX can be prepared by the following method. Formula IX is a representation of Formula A wherein A is -CH₂- or 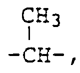 D is 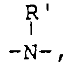 E is 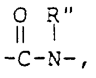 and (Alk₁), (Alk₂), (Alk₃) R', R", R₁ and R₂ are defined as above in Formula A.

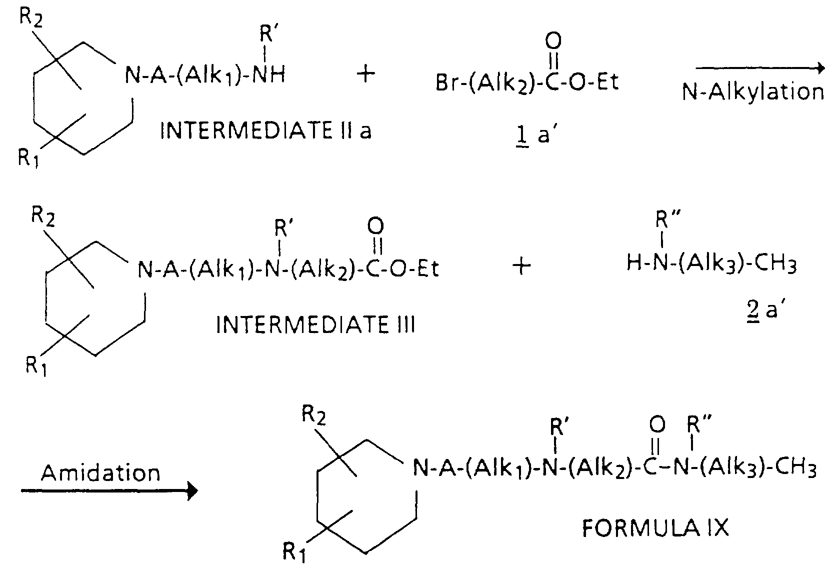

The first step in Reaction Scheme IX is the N-alkylation of the N-substituted piperidine represented by Intermediate IIa (see Reaction Scheme IVA) by the bromoalkyl ester 1a' The appropriate starting materials are a bromoalkyl ester 1a' in which (Alk₂) has the same definition as that desired in the final product and a substituted piperidine (Intermediate IIa) in which R', A and (Alk₁) have the same definitions as that desired in the final product. The bromoalkyl ester 1a' is either commercially available or is readily prepared by techniques well-known in the art.

The alkylation reaction can be conducted utilizing techniques well-known in the art. Typically the bromoalkyl ester 1a' and Intermediate IIa are mixed in approximately a 1:2 molar ratio in a solvent, such as benzene, and the reaction mixture heated at reflux under an inert atmosphere for several hours. Alternatively, the bromoalkyl ester 1a' Intermediate IIa and triethylamine may be mixed in equimolar amounts. Intermediate III is recovered from the reaction mixture and purified by techniques known in the art. For example, the reaction mixture is concentrated under reduced pressure and then taken up in ether and filtered to give the Intermediate III as shown.

The next step in the reaction scheme is an amidation reaction between Intermediate III and a substituted amine as shown in structure 2a'. The substituted amine 2a' chosen is one in which R" and (Alk₃) have the same definition as desired in the final product, represented by Formula IX. The substituted amine 2a' is either commercially available or is readily prepared by techniques well-known in the art. The substituted amine 2a' and Intermediate III are contacted in approximately a 1:1 to a 2:1 molar ratio in the presence of 2-hydroxypyridine and heated at 60°C for several hours. The resulting product can be extracted and purified by techniques well-known in the art. The solution is poured into water, extracted with ethyl acetate, dried over magnesium sulfate and concentrated under reduced pressure. The final product is further purified using chromatographic techniques well-known in the art such as flash chromatography.

### REACTION SCHEME X

A compound according to Formula IX, as prepared and defined above, can be converted to the corresponding amidine by reacting it with triethyloxonium tetrafluoroborate, followed by an amine of the structure R'''-NH₂ to form a compound according to Formula X. 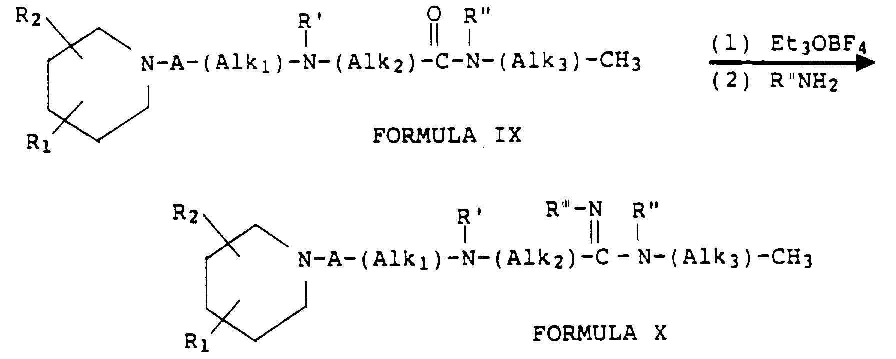 Formula X is a representation of Formula A wherein A is -CH₂ or 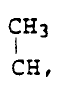 D is 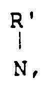 E is 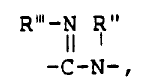 and (Alk₁), (Alk₂), (Alk₃), R', R", R''', R₁ and R₂ are defined as above in Formula A. The amine is chosen so that R''' has the same definition as the definition of R''' desired in the final product. An amide prepared according Reaction Scheme IX is dissolved in methylene chloride and treated with an approximately equimolar amount of triethyloxonium tetrafluoroborate, followed by an excess amount of the desired substituted amine. The final product according to Formula X can be purified by techniques well-known in the art.

Similarly, other complex compounds according to the invention can be made by the following methods.

### REACTION SCHEME XI

Compounds according to Formula XI can be made by following the procedure of Reaction Scheme XI. Formula XI is a representation of Formula A wherein A is 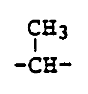 or -CH₂-, D is 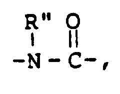 E is 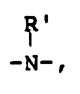 Alk₁, Alk₂, Alk₃, R', R", R₁ and R₂ are defined as above in Formula A.

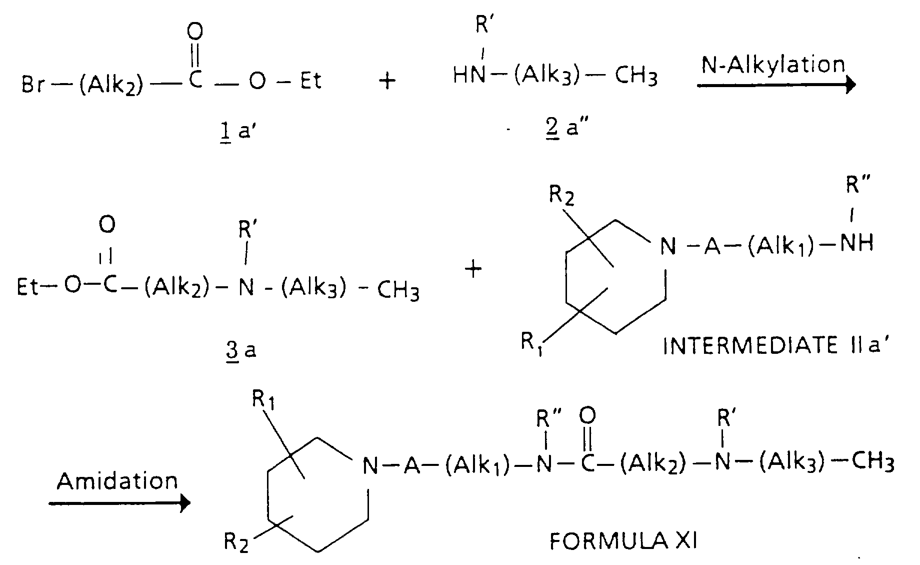

The first step in the reaction sequence is the N-alkylation of an appropriately substituted amine 2a" by bromoalkyl ester 1a'. The appropriate starting materials are a bromoalkyl ester 1a' in which (Alk₂) has the same definition as that desired in the final product and a substituted alkyl amine 2a" in which R' and (Alk₃) have the same definitions as that desired in the final product. The compounds 1a' and 2a" are either commercially available or are readily prepared by techniques well-known in the art. The alkylation reaction can be performed by techniques well-known in the art. Typically the bromoalkyl ester 1a' dans the substituted amine 2a" are mixed in approximately a 1:2 molar ratio in a solvent, such as benzene, and the reaction mixture is heated at reflux under an inert atmosphere for several hours. Alternatively, the bromoalkyl ester 1a', the substituted alkylamine 2a", and triethylamine may be mixed in equimolar amounts. The alkylamine ester 3a is then recovered from the reaction mixture and purified by techniques well-known in the art. For example, the reaction mixture is concentrated under reduced pressure and then taken up in ether and filtered to give the alkylamine ester 3a as shown.

The next step in the reaction scheme is an amidation reaction between the alkylamine ester 3a and Intermediate IIa'. The Intermediate IIa' (see Reaction Scheme IVA) chosen is one in which R₁, R₂, R" and (Alk₁) have the same definition as that desired in the final product, represented by Formula XI. Intermediate IIa' and the alkylamine ester 3a are contacted in approximately a 1:1 to a 2:1 molar ratio in the presence of 2-hydroxypyridine and heated at 60°C for approximately 72 hours, to yield a compound according to Formula XI.

The final product can then be extracted and purified according to methods well-known in the art. The solution is poured into water, extracted with ethyl acetate, dried over magnesium sulfate and concentrated under reduced pressure. The final product is further purified using chromatographic techniques well known in the art such as flash chromatography.

### REACTION SCHEME XII

An amide according to Formula XI, as prepared and defined above, can be reacted with an amine of the structure H₂NR''' to form a compound according to Formula XII. 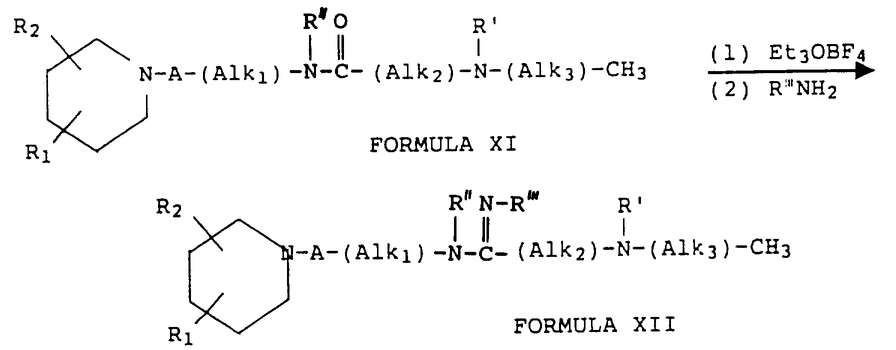 Formula XII is a representation of Formula A wherein D is 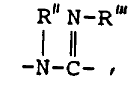 E is 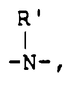 A is -CH₂- or 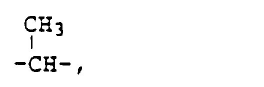 Alk₃ is an alkylene moiety of 0 carbon atoms, and (Alk₁), (Alk₂), R₁, R₂, and R" are defined as in Formula A.

The amine is chosen so that R''' has the same definition as that desired in the final product. An amide prepared according to Reaction Scheme XI is dissolved in methylene chloride and treated with an approximately equimolar amount of triethyloxonium tetrafluoroborate followed by an excess amount of the desired substituted amine. Typically, the reaction is stirred overnight at room temperature followed by heating the reaction to reflux. The solution is then cooled to room temperature, quenched with aqueous sodium hydroxide, dried over a drying agent such as magnesium sulfate and concentrated under reduced pressure.

The resulting oil can be purified by techniques well-known in the art. The resulting oil can by dissolved in ether, filtered and treated with anhydrous hydrochloric acid. The resulting precipitate can be filtered and recrystallized in ethyl acetate/isopropyl alcohol to give the final product according to Formula XII.

Compounds according to Formulas XIII and XIV can be made by the following reaction schemes.

### REACTION SCHEME XIII

A mixed amine and amide according to Formula XIII can be made by the following method. Formula XIII is a representation of Formula A wherein A is -CH₂- or 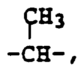 D is 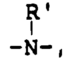 E is 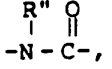 and (Alk₁), (Alk₂), (Alk₃), R', R", R₁ and R₂ are defined as above in Formula A.

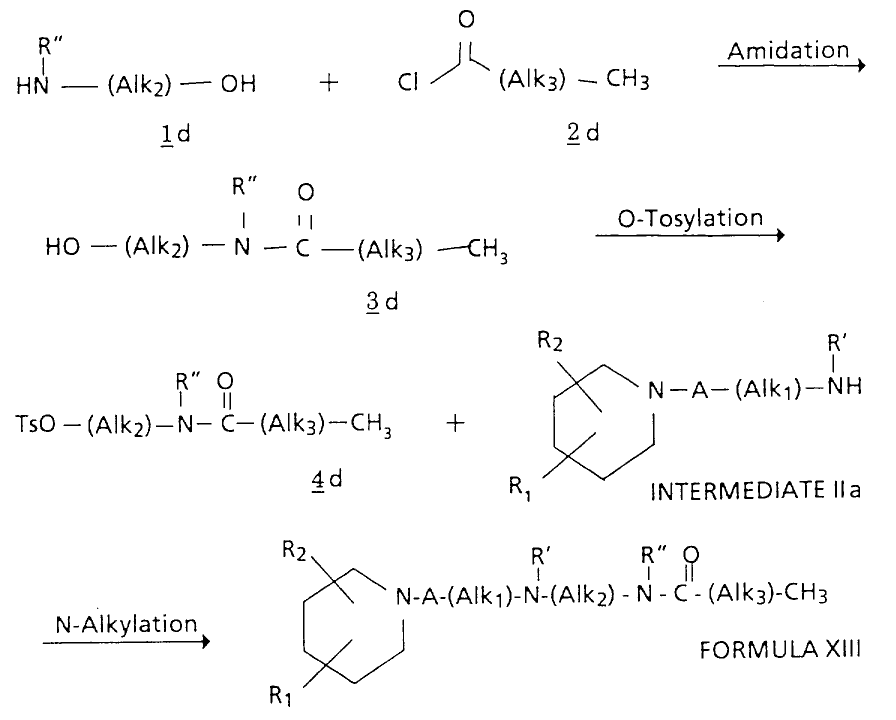

The first step in the reaction is the amidation of acid chloride 2d with amino alcohol 1d. The acid chloride 2d and the amino alcohol 1d are chosen so that R", Alk₂ and Alk₃ have the same definitions as desired in the final product. The compounds 1d and 2d are either commercially available or are readily prepared by techniques well-known in the art. The reactants are mixed in approximately equimolar amounts in the presence of 1 equivalent of triethylamine in methylene chloride at 0-10°C under inert atmosphere. The resulting product 3d is then recovered from the reaction mixture and purified by techniques known in the art, flash chromatography for example.

The second step in the reaction scheme is the O-tosylation of alcohol 3d to provide tosylate 4d, by techniques well-known in the art.

The third step in the reaction scheme is the N-alkylation of the tosylate 4d by Intermediate IIa (see Reaction Scheme IVA). The Intermediate IIa is chosen so that R₁, R₂, A, Alk₁ and R' have the same definitions as desired in the final product. The reactants are mixed in approximately a 1:1 to a 1:2 molar ratio in a solvent, such as benzene, and the reaction heated at reflux under an inert atmosphere for approximately 16 hours. The resulting product can be purified by techniques well-known in the art. The resulting oil can be dissolved in ether, washed with aqueous sodium hydroxide, filtered, and treated with anhydrous hydrochloric acid. The resulting precipitate can be filtered and recrystallized in ethyl acetate/isopropyl alcohol, for example, to give the final product according to Formula XIII, as the hydrochloride salt.

### REACTION SCHEME XIV

A compound according to Formula XIII, defined and prepared as above, can be reacted with triethyloxonium tetrafluoroborate and an amine of the structure H₂NR''' to form a compound according to Formula XIV. 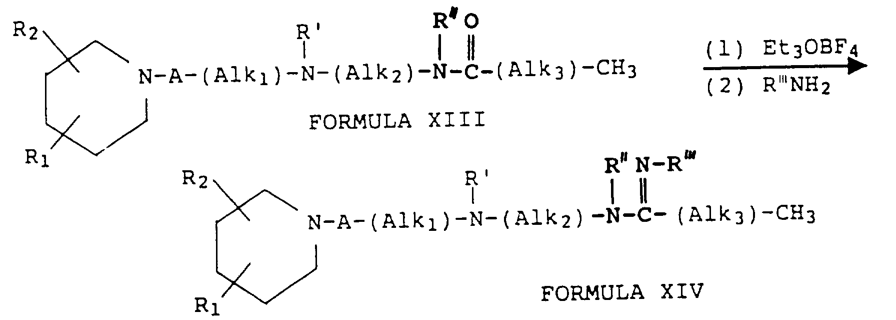 Formula XIV is a representation of Formula A wherein A is -CH₂- or 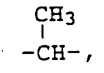 D is 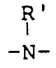 and E is 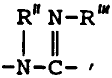 and (Alk₁), (Alk₂), (Alk₃), R₁, R₂, R', R" and R''' are defined as above. The amine H₂NR''' is chosen such that R''' has the same definition as that desired in the final product, represented by Formula XIV.

An amide prepared according Reaction Scheme XIII is dissolved in methylene chloride and treated with an approximately equimolar amount of triethyloxonium tetrafluoroborate followed by an excess amount of the desired substituted amine. The reaction is stirred overnight at room temperature followed by heating the reaction to reflux.

The resulting product can be extracted and purified by techniques well known in the art. The solution is then cooled to room temperature, quenched with sodium hydroxide, dried over a drying agent such as magnesium sulfate and concentrated under reduced pressure. The resulting oil can be dissolved in ether, filtered and treated with anhydrous hydrochloric acid. The resulting precipitate can be filtered and recrystallized in ethyl acetate/isopropyl alcohol to give the final product according to Formula XIV.

### REACTION SCHEME XV

Compounds according to Formula XV can be prepared according to the following reaction scheme. Formula XV is a representation of Formula A wherein A is 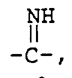 both D and E represent direct bonds and (Alk₁), (Alk₂) and (Alk₃) are represented by (Alkₓ), in which x is an integer from 1 to 18.

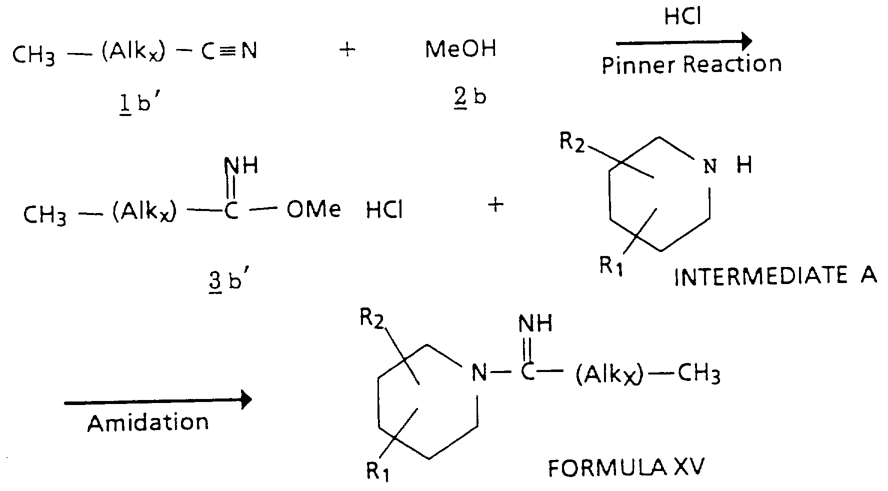

The first step in the reaction sequence is to conduct a Pinner reaction between an alkyl cyanide 1b' and an appropriate alcohol such as methanol 2b. The appropriate starting material is an alkyl cyanide 1b' in which Alkₓ has the same definition as that desired in the final product. The compound 1b' is either commercially available or is readily prepared from available materials by techniques generally known in the art.

The Pinner reaction can be conducted utilizing techniques well-known in the art. Typically, approximately equimolar amounts of alkyl cyanide 1b' and an appropriate alcohol 2 are contacted in a solvent such as ether. The reagents are mixed and cooled to about 0°C, followed by the introduction of an acid, such as hydrochloric acid, until saturation. The reaction mixture is stirred overnight.

The imidate ester hydrochloride 3b' produced via the above reaction can be recovered from the reaction mixture and purified by techniques well-known in the art. For example, the resulting precipitate is concentrated under reduced pressure, triturated with ether, filtered and air dried to give the imidate ester 3b' as the hydrochloride salt.

The second step of the reaction scheme is to conduct an amidation reaction. The above product is mixed with an approximately equimolar amount of Intermediate A, from Reaction Scheme A, in methanol and allowed to stand at room temperature overnight.

The final product can be recovered and purified by techniques well-known in the art. For example, the reaction is concentrated under reduced pressure, triturated with ether and the resulting solid product collected by vacuum filtration, washed with ether and air dried to give the final product according to Formula XV.

### REACTION SCHEME XVI

Compounds according to Formula XVI can be made by the following method. Formula XVI is a representation of Formula A wherein A is -CHX, wherein X is hydrogen or -CH₃, and D and E are direct bonds. 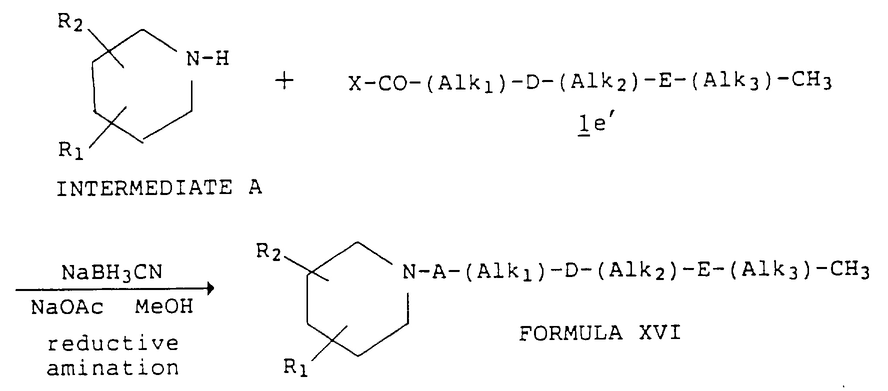 Intermediate A is prepared as described in detail above in Reaction Scheme A. Intermediate A is chosen such that R₁ and R₂ have the same definitions as that desired in the final product. The aldehyde or ketone 1e' is chosen such that X, (Alk₁), (Alk₂) and (Alk₃) have the same definitions as that desired in the final product. The compound 1e' is either commercially available or is readily prepared from available materials by techniques generally known in the art.

Intermediate A is treated with hydrochloric acid in methanol and the solvent removed *in vacuo.* The resulting hydrochloride salt is combined with sodium acetate in approximately a 1:2 ratio together with 4°A molecular sieves and approximately an equimolar amount of the desired aldehyde or ketone 1e'. Bromocresol green (1 mg) in dry methanol is added and the mixture warmed to near reflux for about 1 hour and then cooled, followed by the addition of sodium cyanoborohydride in about a 2:1 ratio to Intermediate A. The resulting solution is refluxed until thin layer chromatography (TLC) shows the reaction is complete.

The desired product according to Formula XVI can be extracted and purified according to methods well-known in the art. For example, the solvent is removed in vacuo and the residue diluted with 10% sodium hydroxide. The aqueous layer is extracted with ether and the extracts dried over magnesium sulfate. The product can be purified by chromatographic methods well-known in the art.

### REACTION SCHEME XVII

Compounds according to Formula XVII can be made by the following method. Formula XVII is a representation of Formula A wherein A is -CH(CF₃)-, and R₁, R₂, R', R", R''', D. E, (Alk₁), Alk₂) and (Alk₃) are all defined as above in Formula A.

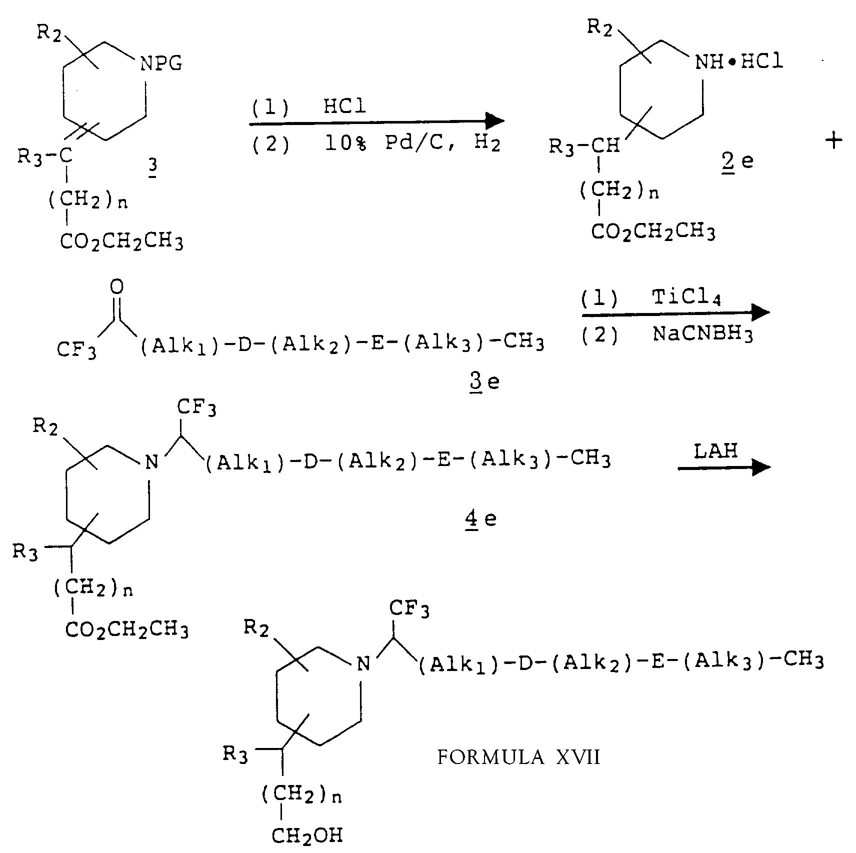

First, the 3- or 4-substituted piperidinylidene propionate 3 is prepared as described in detail above in Reaction Scheme A (compound 3 as depicted on page 5 above). The protecting group (PG) is removed and the olefin reduced by first treating the substituted piperidine 3 with an appropriate acid, such as a saturated solution of hydrochloric acid and the solvent is then removed *in vacuo.* The resulting residue is dissolved in dry ethanol and transferred to a hydrogenation flask. A catalytic amount of 10% Palladium (10% Pd/C) is added and the vessel is charged with hydrogen to about 344.6 kPa (50 psi). After shaking for several hours, the catalyst is filtered through Celite, for example, and the solvent removed *in vacuo* to give the 3- or 4-substituted piperidinyl propionate hydrochloride 2e. Next, the 3- or 4-substituted piperidinyl propionate hydrochloride 2e is reacted with a trifluoromethyl ketone, such as that depicted by 3e. The trifluoromethyl ketone 3e is prepared as depicted in Reaction Scheme XVIIA below.

### REACTION SCHEME XVIIA

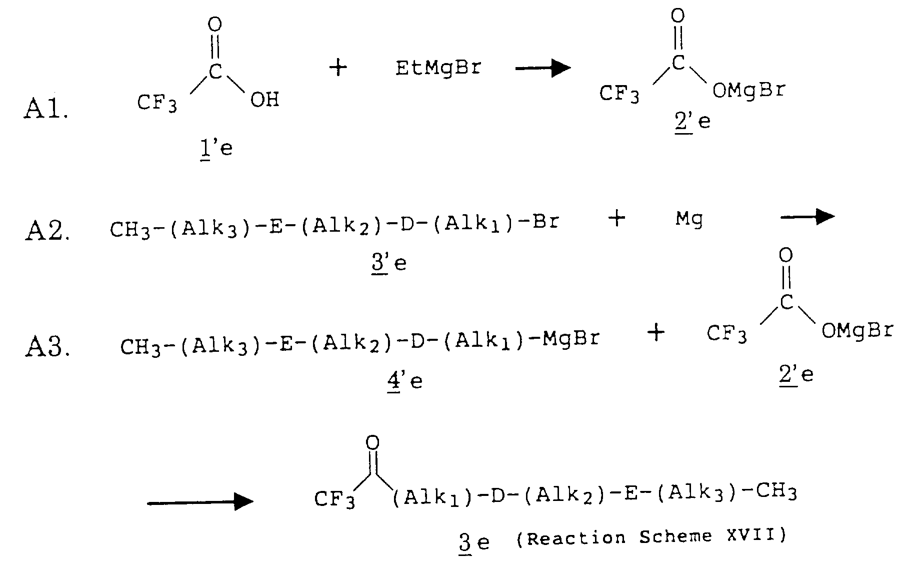

In Reaction Scheme XVIIA, (Alk₁), (Alk₂), (Alk₃), D and E are defined as above in Formula A, with the proviso that D or E is not

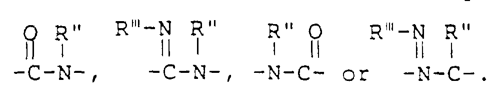

First, magnesium bromide trifluoroacetate 2'e is prepared according to Reaction Scheme XVIIA-A1. To a solution of trifluoroacetic acid 1'e dissolved in an appropriate organic solvent, such as anhydrous ether, is added an approximately equimolar amount of a Grignard reagent, such as ethylmagnesium bromide (EtMgBr), in solution in anhydrous ether at low temperature (-5°C) under a nitrogen atmosphere. The reaction is then allowed to warm to room temperature.

Next, the desired alkylmagnesium bromide 4'e is prepared according to Reaction Scheme XVIIA-A2. The appropriate alkylbromide 3'e is chosen such that (Alk₁), (Alk₂), (Alk₃), D and E are all defined the same as that desired in the product 4'e. The alkylbromide 3'e is either known in the art or is prepared by methods generally known in the art. The alkylbromide 3'e in solution with anhydrous ether is added to magnesium in anhydrous ether (equimolar amounts of alkylbromide 3'e and magnesium). The reaction is stirred at room temperature until the magnesium is dissolved.

To the flask containing the magnesium bromide trifluoroacetate 2'e is added the alkylmagnesium bromide 4'e, in approximately equimolar amounts, at low temperature (-5°C) under a nitrogen atmosphere. The reaction is stirred for about 1 hour at room temperature, refluxed for several hours, cooled to about 0°C and then hydrolyzed by the dropwise addition of 5N hydrochloric acid, for example. The layers are then separated, the aqueous extracted with ethyl acetate, the combined organic extracts are washed with cold saturated sodium bicarbonate solution, and dried. Evaporation yields an oil which can be purified by distillation to yield the trifluoromethylalkyl ketone 3e according to Reaction Scheme XVIIA. The trifluoromethylalkyl ketone 3e and the substituted piperidine 2e are then mixed in approximately equimolar amounts in the presence of an excess of triethylamine and anhydrous methylene chloride at about 10°C under nitrogen atmosphere. Titanium tetrachloride is added dropwise over about 10 minutes in a molar amount approximately half that of the molar amount of the trifluoromethylalkyl ketone 3e. The reaction is stirred at room temperature for approximately 48 hours, then carefully quenched with a methanolic solution of excess sodium cyanoborohydride. The reaction is made acidic with 5N hydrochloric acid, then made basic with 5N sodium hydroxide, for example. The desired product is extracted with ethyl acetate, dried over magnesium sulfate and evaporated, providing the trifluoromethylalkyl substituted piperidine ester 4e.

Finally, the trifluoromethylalkyl substituted piperidine ester 4e is reduced to the corresponding alcohol XVII To an ice cooled, stirred solution of the trifluoromethylalkyl substituted piperidine ester 4e in dry THF is added an excess of diisopropylaluminum hydride in toluene. The resulting mixture is stirred for an approximately 1 hour and carefully quenched with methanol (0°C). The solution is then diluted with ether and washed with 10% sodium hydroxide. The resulting emulsion is filtered through Celite, the organic layer separated, dried over magnesium sulfate and the solvent removed *in vacuo* to give the product XVII.

As examples of compounds of the present invention are the following:
1. 4-(2-Hydroxy-1-methylethyl)-N-(3-methylbutyI)-1-piperidinebutanamide.
2. β-Methyl-1-(4-[(3-methylbutyl)amino]butyl]-4-piperidineethanol.
3. 1-(1-Imino-4-methylpentyl)-β-methyl-4-piperidine-ethanol.
4. N-Butyl-4-(2-hydroxy-1-methylethyl)-N-methyl-1-piperidinebutanamide.
5. 4-(2-Hydroxy-l-methylethyl)-1-piperidinebutanenitrile.
6. 1-(4-Aminobutyl)-β-methyl-4-piperidineethanol.
7. N-[4-[4-(2-Hydroxy-1-methylethyl)-1-piperidinyl] butyl]-4-methylpentanimidamide.
8. 1-(1-Iminooctyl)-β-methyl-4-piperidineethanol.
9. 1-(1,5,9-Trimethyldecyl)-β-methyl-4-piperidineethanol.
10. 2-[1-(1-Trifluoromethyl)undecyl-4-piperidinyl]-propanol.

The following assays are used to test compounds for their ability to inhibit 2,3-oxidosqualene lanosterol-cyclase or epoxidase. Microsomes, prepared by ultracentrifugation of homogenates of rat liver, are incubated at 37°C for 45 minutes in the presence of 60 µM 3H-squalene, 2.0 mM NADPH, 0.01 mM FAD, and the high speed supernatant fraction from the microsomal preparation. Blanks, in which NADPH has been omitted, are run simultaneously with the test compounds. Compounds are tested at concentrations of >0.0 to 100.0 µM.

### Method 1

Following incubation the samples are saponified, standards are added to each sample, and then the reaction products are extracted into hexane. The hexane extracts are dried and then the dried extracts are redissolved in chloroform. The reaction products contained in the extracts are then separated by TLC. Spots containing the reaction products are scraped from the TLC plates and counted for radioactivity in a scintillation counter. An IC₅₀ is finally calculated.

### Method 2

Following incubation reactions are stopped by the addition of chloroform:methanol, standards are added, then reaction products and standards are extracted into chlorolorm. The chloroform extracts are dried, and the residue is dissolved in toluene:methanol. The reaction products and standards contained in the dissolved residue are separated by high performance liquid chromatography (HPLC). Chromatographic peaks containing reaction products are monitored for radioactivity with a flow-through scintillation counter connected in series with the HPLC column. An IC₅₀ is calculated based on the radioactivity in controls and samples.

### Pharmaceutical Preparations of the 2,3-Oxidosqualene Lanosterol-Cyclase Inhibitors

The compounds of this invention are useful both in the free base form and in the form of acid addition salts. The acid addition salts are simply a more convenient form for use and, in practice, use of the salt amounts to use of the free base. The expression "pharmaceutically acceptable acid addition salts" is intended to apply to any non-toxic organic or inorganic acid addition salts of the base compounds of the above compounds. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the sulfonic acids such as p-toluenesulfonic, methanesulfonic acid and 2-hydroxyethanesulfonic acid. Either the mono- or the di-acid salts can be formed, and such salts can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solvent, or by reacting the free base in an organic solvent in which case the salt separates directly or can be obtained by concentration of the solution.

The preferred route of administration is oral administration. For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures.

The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The exact amount of the compound or compounds to be employed, i.e., the amount of the subject compound or compounds sufficient to provide the desired effect, depends on various factors such as the compound employed; type of administration; the size, age and species of animal; the route, time and frequency of administration; and, the physiological effect desired. In particular cases, the amount to be administered can be ascertained by conventional range finding techniques.

The compounds are preferably administered in the form of a composition comprising the compound in admixture with a pharmaceutically acceptable carrier, i.e., a carrier which is chemically inert to the active compound and which has no detrimental side effects or toxicity under the conditions of use. Such compositions can contain from about 0.1 µg or less to 500 mg of the active compound per ml of carrier to about 99% by weight of the active compound in combination with a pharmaceutically-acceptable carrier.

The compounds may also be incorporated into any inert carrier so that they may be utilized in routine serum assays, blood levels, urine levels, etc., according to techniques well known in the art.

The compositions can be in solid forms, such as tablets, capsules, granulations, feed mixes, feed supplements and concentrates, powders, granules or the like; as well as liquid forms such as sterile injectable suspensions, orally administered suspensions or solutions. The pharmaceutically acceptable carriers can include excipients such as surface active dispersing agents, suspending agents, tableting binders, lubricants, flavors and colorants. Suitable excipients are disclosed, for example, in texts such as *Remington's Pharmaceutical Manufacturing*, 13 Ed., Mack Publishing Co., Easton, Pennsylvania (1965).

The following examples are presented to illustrate the present invention but they should not be construed as limiting in any way.

### EXAMPLE 1a

### PREPARATION OF 2-[1-(PHENYLMETHYL)-4-PIPERIDINYLIDENE PROPANOIC ACID-ETHYL ESTER

To a stirred solution of triethyl 2-phosphonopropionate (5.72 g, 24 mmol) in 250 ml of anhydrous tetrahydrofuran at -78°C under argon, was added a solution of n-butyllithium in hexane (16.3 ml, 26 mmol). The resulting anion was stirred for an additional 10 minutes at which time N-benzyl-4-piperidinone (3.79 g, 20 mmol) was added in 50 ml of tetrahydrofuran dropwise via syringe. The mixture was subsequently stirred for 10 minutes, warmed to room temperature, and stirred for an additional 17 hours. The solution was then diluted with 100 ml of a saturated solution of ammonium chloride, washed twice with 10% sodium hydroxide, dried over magnesium sulfate and evaporated to give 6.58 g of a crude oil which was purified by chromatography using a 25% ethyl acetate/hexane solution as the eluent. Removal of the solvent *in vacuo* gave 5.25 g (96% yield) of a colorless oil that was identified as 2-[1-(phenylmethyl)-4-piperidinylidene]propanoic acid ethyl ester on the basis of the following spectral data: MS, CI/CH₄; m/z 274 (M+H)(base), 228 (M+H-EtOH), 196 (M+H-C₆H₆).

### EXAMPLE 1b

### PREPARATION OF 2-[1-(PHENYLMETHYL)-4-PIPERIDINYLIDENE]PROPANOL

To an ice cooled, stirred solution of 550 mg (2.0 mmol) of 2-[1-(phenylmethyl)-4-piperidinylidene]propanoic acid ethyl ester in 50 ml of dry tetrahydrofuran was added 4.0 ml (6.0 mmol, 1.5 M) of diisopropylaluminum hydride in toluene. The resulting mixture was stirred for an additional hour and carefully quenched with methanol at 0°C. The solution was then diluted with ether and washed with 10% sodium hydroxide. The resulting emulsion was filtered through Celite, the organic layer separated, dried over magnesium sulfate and the solvent removed by rotary evaporation to give 460 mg of an oil that was identified as 2-[1-(phenylmethyl)-4-piperidinylidene]propanol on the basis of the following spectral data: HRMS calculated for C₁₅H₂₁NO: 232.1701; Found: 232.1699.

### EXAMPLE 2

### PREPARATION OF 1-1,5,9-TRIMETHYLDECYL)-β-METHYL-4-PIPERIDINEETHANOL

A 300 mg (12.2 mmol) sample of 2-[l-(phenylmethyl)-4-piperidinylidene] propanoic acid-ethyl ester was treated with a saturated solution of hydrochloric acid in methanol (HCl/CH₃OH) and the solvent removed by rotary evaporation. The resulting residue was dissolved in 25 ml of dry ethanol and transferred to a Parr hydrogenation flask. A catalytic amount of 10% Palladium(C) (101 mg) was added and the vessel charged with hydrogen to 344.6 kPa (50 psi). After shaking for 37 hours, the catalyst was filtered through Celite and the solvent removed *in vacuo.* The resulting residue was again treated with HCl/CH₃OH and the solvent removed *in vacuo.* The resulting hydrochloride salt was combined with sodium acetate (180 mg, 2.2 mmol), 4Å molecular sieves (2.0 g), and 6,10-dimethyl-2-undecanone (300 mg, 1.5 mmol) and 1 mg of bromocresol green in 20 ml of dry methanol. The mixture was warmed near reflux for 1 hour, then cooled and sodium cyanoborohydride (138 mg, 2.2 mmol) added. The resulting solution was refluxed for 30 hours at which time thin layer chromatography analysis showed complete reaction. The solvent was removed *in vacuo* and the residue diluted with 10% sodium hydroxide (20 ml). The aqueous layer was extracted with three portions of ether (30 ml) and the combined extracts were dried over magnesium sulfate. Removal of the solvent *in* *vacuo* gave an oil which was dissolved in-20 ml of dry tetrahydrofuran and treated with excess lithium aluminum hydride (10 ml, 1.0 M solution in ether). After 30 minutes, the reaction was quenched according to the method of Fieser & Fieser, *supra,* and the solvent removed to give an oil that was purified by chromatography on silica gel, using first 25% ethyl acetate: hexane solution followed by a 20% methanol:chloroform solution as the eluents. Concentration of the methanol: chloroform fractions gave 247 mg (69% yield) of an oil that was identified as 1-(1,5,9-trimethyldecyl)-β-methyl-4-piperidineethanol on the basis of the following spectral data: MS, EI/70eV; m/z 325 (M+), 310 (M-CH₃), 170 (base).

### EXAMPLE 3

### PREPARATION OF β-METHYL-4-PIPERIDINEETHANOL

To a solution of 1.5 g (5.5 mmol) of 2-[1-(phenylmethyl)-4-piperidinylidene] propanoic acid ethyl ester in 50 ml of acetic acid in a Parr hydrogenation flask, was added 500 mg of 10% palladium(C). The vessel was charged with hydrogen to 344.6 kPa (50 psi). After shaking for 18 hours, the solution was filtered through Celite and the solvent removed *in vacuo.* The resulting acetate salt was dissolved in 50 ml of dichloromethane in a 250 ml round bottom flask. To this solution was added 1.33 g (6.1 mmol) of di-5-butyldicarbonate and triethylamine (5 ml). After stirring for 30 minutes at room temperature, the solvent was removed and the residue dissolved in ether (100 ml). The ether was washed with 2N HCl and dried over magnesium sulfate. Removal of the solvent and chromatography on silica gel using 10% ethyl acetate/hexane gave 1.57 g of the carbamate. This material was immediately dissolved in tetrahydrofuran (50 ml) and treated with 12.0 ml (12.0 mmol) of diisobutylaluminum hydride at 0°C under nitrogen. The solution was warmed to room temperature and allowed to stir for 15 hours. The mixture was then cooled to 5°C and quenched by the careful addition of methanol. The resulting emulsion was diluted with ether (100 ml) and filtered through Celite. Removal of the solvent gave 960 mg of the protected alcohol (Ms, CI/CH₄ m/z [M+H]⁺ 244).

The above alcohol was cooled to 5°C and dissolved in 25 ml of trifluoroacetic acid. After the evolution of carbon dioxide was complete, the solvent was removed, the residue dissolved in ether (100 ml) and washed with 10% sodium hydroxide. Continuous ether extraction of the basic layer gave 247 mg of β-methyl-4-piperidineethanol which was identified on the basis of the following spectral data: MS, CI/CH₄ m/z [M+H]+ 144 (base).

### EXAMPLE 4

### PREPARATION OF 4-(2-HYDROXY-1-METHYLETHYL)-N-(3-METHYLBUTYL)-1-PIPERIDINEBUTANAMIDE HYDROCHLORIDE

To a stirred solution containing 995 mg (5.1 mmol) of ethyl 4-bromobutyroate and 730 mg (5.1 mmol) of β-methyl-4-piperidineethanol in 50 ml of dry benzene is added 840 µl (6.1 mmol) of triethylamine. The mixture is heated at reflux under a nitrogen atmosphere overnight. The solution is concentrated under reduced pressure, ether added and filtered. The filtrate is washed with 10% sodium hydroxide, the organic layer collected, dried over magnesium sulfate and evaporated to give 4-(2-hydroxy-1-methylethyl)-1-piperidinebutanoic acid ethyl ester as the product.

The 4-(2-hydroxy-1-methylethyl)-1-piperidinebutanoic acid ethyl ester (1.0 g, 3.9 mmol) is then combined with 850 mg (9.8 mmol) of isopentylamine and 367 mg (3.86 mmol) of 2-hydroxypyridine and heated together at 60°C in a sealed tube. After the reaction was judged complete (gas chromatography), the solution is poured into water, extracted with ethyl acetate, dried over magnesium sulfate and concentrated under reduced pressure to give 4-(2-hydroxy-1-methylethyl)-N-(3-methylbutyl)-1-piperidinebutanamide as the product. This material is dissolved in ether and treated with anhydrous hydrochloric acid to give, after removal of the solvent, a solid which is purified by recrystallization to give 4-(2-hydroxy-1-methylethyl)-N-(3-methylbutyl)-1-piperidinebutanamide hydrochloride as the product.

### EXAMPLE 5

### PREPARATION OF β-METHYL-1-[4-[(3-METHYLBUTYL)AMINO]-BUTYL]-4-PIPERIDINEETHANOL HYDROCHLORIDE

To a stirred solution of 4-(2-hydroxy-1-methylethyl)-N-(3-methylbutyl)-1-piperidinebutanamide (prepared from 1.0 g, 3.3 mmol, or the hydrochloride salt by base extraction) in 100 ml of dry tetrahydrofuran at 0°C under nitrogen is added a solution of lithium aluminum hydride (4.0 ml, 1.0 M) in ether dropwise via syringe. The resulting mixture is stirred at room temperature overnight and subsequently heated at reflux. The solution is then cooled and the reaction stopped by the addition of water and a 15% solution of sodium hydroxide. The precipitate is filtered and the solvent evaporated to give the product. This material is dissolved in ether and treated with anhydrous hydrochloric acid to give, after removal of the solvent, a solid which is purified by recrystallization to give β-methyl-1-[4-[(3-methylbutyl)amino]butyl]-4-piperidineethanol hydrochloride as the product.

### EXAMPLE 6

### PREPARATION OF 1-(1-IMINO-4-METHYLPENTYL)-β-METHYL-4-PIPERIDINEETHANOL HYDROCHLORIDE

First, 4-methylvaleronitrile (10.0 g, 0.103 mol) and methanol (3.5 g, 0.11 mol) are dissolved in ethyl ether (100 ml), cooled to 0°C, saturated with anhydrous hydrochloric acid and allowed to stir overnight. The resulting solution is concentrated under reduced pressure and triturated with ether. The precipitate formed is filtered and air dried to give methyl 4-methyl-pentanimidic acid methyl ester hydrochloride as the product.

A solution containing a mixture of 1.0 g (7.0 mmol) of β-methyl-4-piperidineethanol and 1.16 g (7.0 mmol) of 4-methyl-pentanimidic acid methyl ester hydrochloride in dry methanol is stirred overnight at room temperature, the solvent concentrated and ether added. The resulting precipitate is filtered to give 1-(1-imino-4-methylpentyl)-β-methyl-4-piperidineethanol hydrochloride.

### EXAMPLE 7

### PREPARATION OF N-BUTYL-4-(2-HYDROXY-1-METHYLETHYL)-N-METHYL-1-PIPERIDINEBUTANAMIDE HYDROCHLORIDE

4-(2-Hydroxy-1-methylethyl)-1-piperidinebutanoic acid ethyl ester (1.0 g, 3.9 mmol) is combined with 854 mg (9.8 mmol) of N-methylbutylamine and 367 mg (3.86 mmol) of 2-hydroxypyridine and heated together at 60°C in a sealed tube. After the reaction was judged complete (gas chromatography), the solution is poured into water, extracted with ethylacetate, dried over magnesium sulfate and concentrated under reduced pressure to give the product. This material is dissolved in ether and treated with anhydrous hydrochloric acid to give, after removal of the solvent, a solid which is purified by recrystallization to give N-butyl-4-(2-hydroxy-1-methylethyl)-N-methyl-1-piperidinebutanamide hydrochloride as the product.

### EXAMPLE 8

### PREPARATION OF N-[4-[4-(2-HYDROXY-1-METHYLETHYL)-1-PIPERIDINYL]BUTYL]-4-METHYLPENTANIMIDAMIDE HYDROCHLORIDE

To a stirred solution containing 1.04 g (7.0 mmol) of 4-bromobutyronitrile and 1.0 g (7.0 mmol) of β- methyl-4-piperidineethanol in 50 ml of dry benzene is added 1.15 ml (8.4 mmol) of triethylamine. The mixture is heated at reflux under a nitrogen atmosphere overnight. The solution is concentrated under reduced pressure, ether added and filtered. The filtrate is washed with 10% sodium hydroxide, the organic layer collected, dried over magnesium sulfate and evaporated to give 4-(2-hydroxy-1-methylethyl)-1-piperidinebutanenitrile as the product.

To an ice cooled, stirred solution of 4-(2-hydroxy-1-methylethyl)-1-piperidinebutanenitrile (1.0 g 4.8 mmol) in 50 ml of anhydrous tetrahydrofuran under nitrogen atmosphere is added (5.0 ml, 1.0 M) lithium aluminum hydride in ether dropwise via syringe. The resulting mixture is stirred at room temperature overnight and subsequently heated at reflux. The solution is then cooled-and the reaction stopped by the addition of water and a 15% solution of sodium hydroxide. The precipitate is filtered and the solvent evaporated to give 1-(4-aminobutyl)-β-methyl-4-piperidineethanol as the product.

A solution containing a mixture 1.0 g (4.7 mmol) of 1-(4-aminobutyl)-β-methyl-4-piperidineethanol and 1.16 g (7.0 mmol) of 4-methyl-pentanimidic acid methyl ester hydrochloride in dry methanol is stirred overnight at room temperature, the solvent concentrated and ether added. The resulting precipitate is filtered to give N-[4-[4-(2-hydroxy-1-methylethyl)-1-piperidinyl]butyl]-4-methylpentanimidamide hydrochloride as the product.

### EXAMPLE 9

### PREPARATION OF 1-(1-IMINOOCTYL)-β-METHYL-4-PIPERIDINEETHANOL HYDROCHLORIDE

Octyl cyanide (10.0 g, 0.079 mol) and methanol (2.56 g, 0.08 mol) is dissolved in 50 ml ethyl ether, cooled to 0°C, saturated with anhydrous hydrochloric acid and allowed to stir overnight. The resulting solution is concentrated under reduced pressure and triturated with ether. The precipitate formed is filtered and air dried to give octanimidic acid methyl ester hydrochloride as the product.

A solution containing a mixture of 1.0 g (7.0 mmol) of β-methyl-4-piperidineethanol and 1.36 g (7.0 mmol) of octanimidic acid methyl ester hydrochloride in dry methanol is stirred overnight at room temperature, the solvent concentrated and ether added. The resulting precipitate is filtered and dried to give 1-(1-iminooctyl)-β-methyl-4-piperidineethanol hydrochloride as the product.

### EXAMPLE 10

### PREPARATION OF 2-[1-(1-TRIFLUOROMETHYL)-UNDECYL-4-PIPERIDINYL]PROPANOL

### Preparation of Magnesium Bromide Trifluoroacetate (1)

To a dry flask containing trifluoroacetic acid (15 g, 0.132 mmol) and anhydrous ether (50 ml) was added a solution of ethylmagnesium bromide (66 ml of a 2 M solution in tetrahydrofuran, 0.132 mmol) in anhydrous ether (50 ml) at -5°C under nitrogen atmosphere. The reaction was allowed to warm to room temperature.

### Preparation of Decanylmagnesium Bromide (2)

To a dry flask containing magnesium (2.64 g, 0.11 mmol) and anhydrous ether (50 ml) was added a solution of 1-bromodecane (24.3 g, 0.11 mmol) in anhydrous ether (50 ml) under nitrogen atmosphere. The reaction was stirred at room temperature until the magnesium had dissolved.

### Preparation of Trifluoromethyl-2-dodecanone (3)

To the flask containing magnesium bromide trifluoroacetate (1) was added the solution of decanylmagnesium bromide (2) at -5°C under nitrogen atmosphere. The reaction was stirred for 1 hour at room temperature, refluxed for 12 hours, cooled to 0°C and hydrolyzed with the dropwise addition of 5N hydrochloric acid (50 ml). The layers were separated, the aqueous extracted with ethyl acetate (2 x 20 ml), the combined organics washed with cold saturated sodium bicarbonate solution then brine, and dried over magnesium sulfate. Evaporation gave 14.1 g of yellow oil which was purified by distillation providing trifluoromethyl-2-dodecanone as a clear oil (10.1 g, 38%), b.p. 125°C @ 0.1 mm Hg. ¹H-NMR (300 MHz, CDCl₃) δ 0.85 (3H, t), 1.30 (14H, br s), 1.65 (2H, m), 2.70 (2H, t); ¹⁹F-NMR (CDCl₃) δ -80.02 (s); MS (CI/CH₄) m/z 239 (M+H), 169 (M+H - HCF₃).

### Preparation of Ethyl 2-(4-Piperidinyl)propionate ·HCl (4)

A 300 mg (12.2 mmol) sample of 2-[1-(phenylmethyl)-4-[piperidinylidene] propanoic acid ethyl ester was treated with a saturated solution of hydrochloric acid in methanol and the solvent removed by rotary evaporation. The resulting residue was dissolved in 25 ml of dry ethanol and transferred to a Parr hydrogenation flask. A catalytic amount of 10% Palladium(C) (101 mg) was added and the vessel charged with hydrogen to 344.6 kPa (50 psi). After shaking for 37 hours, the catalyst was filtered through Celite and the solvent removed *in vacuo.* The resulting residue was again treated with hydrochloric acid in methanol and the solvent removed *in vacuo* giving ethyl 2-(4-piperidinyl)propionate hydrochloric acid (4).

### Preparation of 2-[1-(1-Trifluoromethyl)undecyl-4 piperidinyl]propanoic Acid Ethyl Ester (5)

To a dry flask containing trifluoromethyl-2-dodecanone (3) (4.0 g, 16.8 mmol), ethyl 2-(4-piperidinyl)propionate hydrochloride (3.17 g, 15.1 mmol), triethylamine (5 g, 50.4 mmol), and anhydrous methylene chloride (80 ml) at 10°C under nitrogen atmosphere is added titanium tetrachloride (8.4 ml of a 1 M solution in methylene chloride, 8.4 mmol) dropwise over 10 minutes. The reaction is stirred at room temperature for 48 hours, then carefully quenched with a methanolic solution of sodium cyanoborohydride (3.4 g, 50.4 mmol in 20 ml methanol). The reaction is stirred for 1 hour, carefully taken to pH 1 with 5N hydrochloric acid, stirred for 30 minutes, then taken to pH 13 with 5N sodium hydroxide. The desired product is extracted with ethyl acetate (3 x 75 ml), dried over magnesium sulfate, and evaporated providing 2-[1-(1-trifluoromethyl)undecyl-4-piperidinyl]propanoic acid ethyl ester (5).

### Preparation of 2-[1-(1-Trifluoromethyl)undecyl 4-poperidinyl]propanol (6)

To an ice cooled, stirred solution of 2-[1-(1-trifluoromethyl)undecyl-4-piperidinyl propanoic acid ethyl ester (779 mg, 2.0 mmol) in 50 ml of dry tetrahydrofuran is added 4.0 ml (6.0 mmol, 1.5 M) of diisopropylaluminum hydride in toluene. The resulting mixture is stirred for an additional hour and carefully quenched with methanol at 0°C. The solution is then diluted with ether and washed with 10% sodium hydroxide. The resulting emulsion is filtered through Celite, the organic layer separated, dried over magnesium sulfate and the solvent removed by rotary evaporation to give 2-[1-(1-trifluoromethyl)undecyl-4-piperidinyl]-propanol (6).

## Claims

1. A compound according to the formula A: 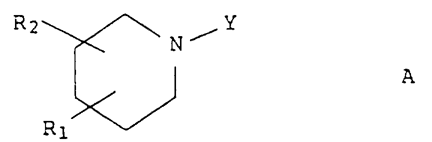 and the pharmaceutically acceptable salts thereof wherein Y is -A-(Alk₁)-D-(Alk₂)-E-(Alk₃)-CH₃ wherein, A is -CH₂-, 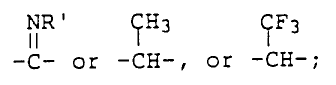 D and E are each independently -CH₂-, 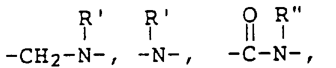 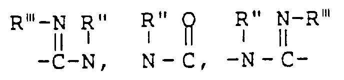 or a direct bond, with the proviso that when D is a moiety from the group 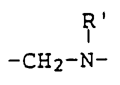 or 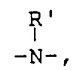 E cannot be a moiety from the same group, and that when D is a moiety form the group 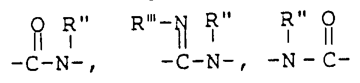 or 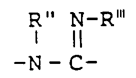 E cannot be a moiety from the same group; and, with the proviso that when A is -CH(CF₃), D and/or E cannot be a moiety from the group 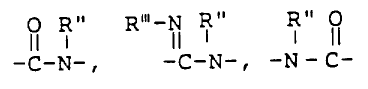 or 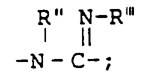
(Alk₁), (Alk₂) and (Alk₃) are each independently a straight chain alkylene moiety containing from 0 to 5 carbon atoms, optionally substituted with from 1 up to 3 methyl groups, with the proviso that (Alk₂) cannot have the value of 0 carbon atoms; or,
(Alk₁), (Alk₂) and (Alk₃) are each independently a scraight chain alkenylene moiety containing from 2 up to 6 carbon atoms, the straight chain alkenylene moiety having 1 to 2 double bonds, and optionally substituted with from 1 up to 3 methyl groups;
R₁ is a group of the formula 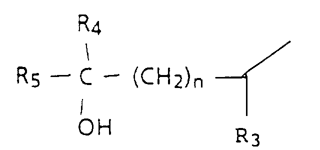
positioned in either the 3 or the 4 position of the piperidine ring, wherein R₃ is (C₁-C₄) lower alkyl, R₄ and R₅ are each independently hydrogen or (C₁-C₄) lower alkyl and n is zero or an integer from 1 to 3;
R₂ is hydrogen or up to 3 independent hydroxy or (C₁-C₄) lower alkyl substituents; and
R', R" and R"' are each independently hydrogen or (C₁-C₄) lower alkyl.

2. A compound according to Claim 1 wherein R₁ is a group of the formula

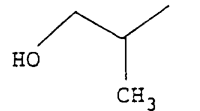

3. A compound according to Claim 1 which is 4-(2-hydroxy-1-methylethyl)-N-(3-methylbutyl)-1-piperdinebutan-amide, β-methyl-1-[4-[(3-methylbutyl)-amino]butyl]-4-piperidineethanol, 1-(1-imino-4-methylpentyl)-β-methyl-4-piperidineethanol, N-butyl-4-(2-hydroxy-1-methylethyl)-N-methyI-1-piperidinebutanamide, N-[4-[4-(2-hydroxy-1-methylethyl)-1-piperidinyl]butyl]-4-methy1-pentanimidamide, 1-(1-iminooctyl)-β-methyl-4-piperidineethanol, 1-(1,5,9-trimethyldecyl)-β-methyl-4-piperidineethanol, 2-[1-(1-trifluoromethyl)undecyl-4-piperidinyl]propanol, and the pharmaceutically acceptable salts thereof.

4. A compound which is 4-(2-hydroxy-1-methylethyl)-1-piperidinebutanenitrile, 1-(4-aminobutyl)-β-methyl-4-piperidineethanol, 2-(1-phenylmethyl)-4-piperidinylidene]-1-propanol, and the pharmaceutically acceptable salts thereof.

5. A composition comprising a compound of any of claims 1 to 3 present in admixture with an inert carrier.

6. A pharmaceutical composition comprising a compound of any of claims 1 to 3 present in admixture with a pharmaceutically acceptable carrier.

7. A pharmaceutical composition according to claim 6 for lowering plasma cholesterol in mammals.

8. A pharmaceutical composition according to claim 6 for treating fungal infections.

9. Use of a compound according to any of claims 1 to 3 in admixture with a pharmaceutically acceptable carrier for the preparation of a pharmaceutical composition.

10. Use according to claim 9 wherein the pharmaceutical composition is effective for lowering plasma cholesterol levels.

11. Use according to claim 9 wherein the pharmaceutical composition is effective for treating fungal infections.

12. A method for the preparation of a pharmaceutical composition as defined in claims 6-8 comprising combining a compound as defined in any one of claims 1-3 with a pharmaceutically acceptable carrier.

13. A method for the preparation of a compound as defined in claims 1 to 3 characterized in that the method comprises at least one reaction according to the following reaction schemes:
REACTION SCHEME I wherein compounds according to Formula I which is a representation of Formula A wherein D is 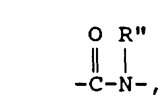 A is -CH₂ or 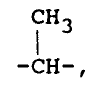 (Alk₃) is an alkylene moiety of 0 carbon atoms, (Alk₁) and (Alk₂) and R" are defined as in Formula A, and E is a direct bond, are prepared by an amidation reaction between Intermediate I and a substituted amine 2a: 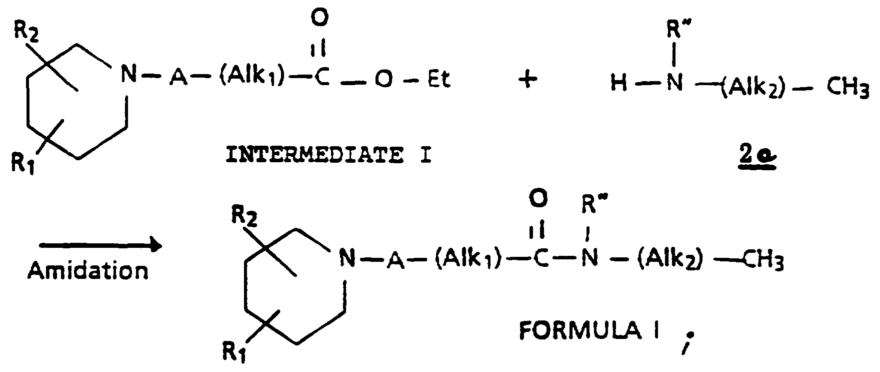 REACTION SCHEME II
wherein an amide according to Formula I is reduced to the corresponding amine of Formula II which is a representation of Formula A wherein D is 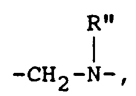 A is -CH₂- or 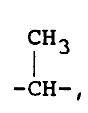 E is a direct bond, (Alk₃) is an alkylene moiety of 0 carbon atoms, and (Alk₁), (Alk₂), R₁, R₂ and R" are defined as in Formula I: 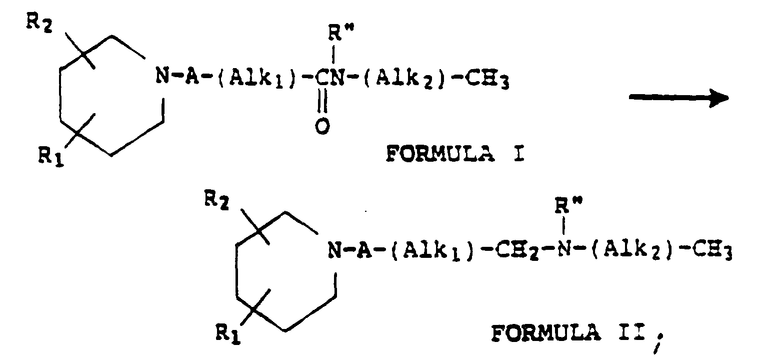 REACTION SCHEME III
wherein the amide according to Formula I is converted to the corresponding amidine according to Formula III which is a representation of Formula A wherein D is 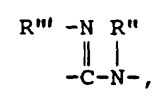 A is -CH₂- or 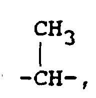 (Alk₃) is an alkylene moiety of 0 carbon atoms, E is a direct bond and (Alk₁), (Alk₂), R₁, R₂, R" and R''' are defined as in Formula A: 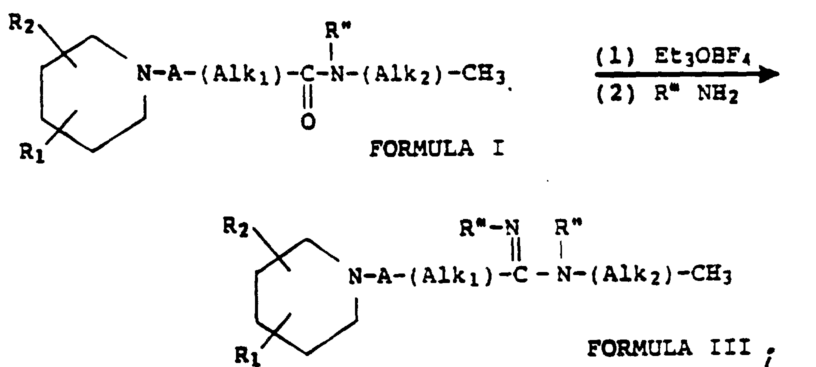 REACTION SCHEME IV
wherein amidines according to Formula IV which is a representation of Formula A wherein D is 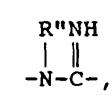 R'' is hydrogen, A is -CH₂- or 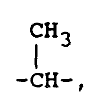 (Alk₃) is an alkylene moiety of 0 carbon atoms, E is a direct bond and (Alk₁), (Alk₂), R₁ and R₂ are defined as in Formula A, is prepared by an amidine formation reaction between Intermediate IIb and the imidate ester 3b: 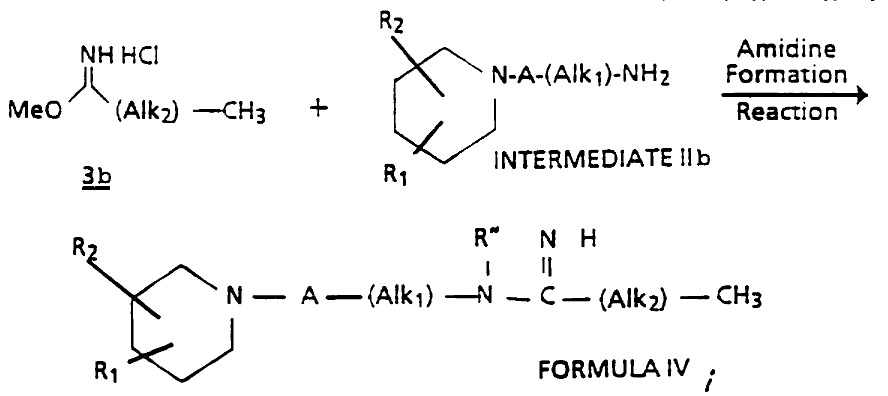 REACTION SCHEME V
wherein the amidine according to Formula IV is hydrolyzed to form an amide according to Formula V which is a representation of Formula A wherein D is 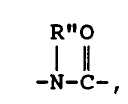 A is -CH₂- or 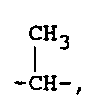 (Alk₃) is an alkylene moiety of 0 carbon atoms, E is a direct bond and (Alk₁), (Alk₂), R₁, R₂ and R" are defined as in Formula A. 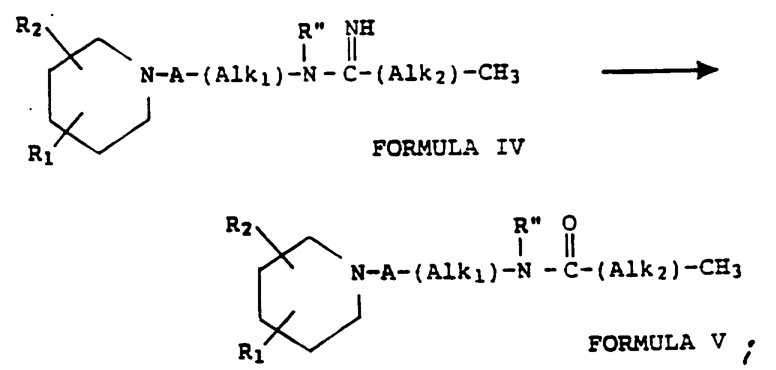 REACTION SCHEME VI
wherein the amide of Formula V is converted to a substituted amidine according to Formula VI which is a representation of Formula A wherein D is 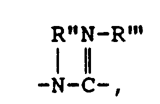 A is -CH₂- or 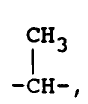 A (Alk₃) is an alkylene moiety of 0 carbon atoms, E is a direct bond and (Alk₁), (Alk₂), R₁, R₂ R" and R''' are defined as in Formula A: 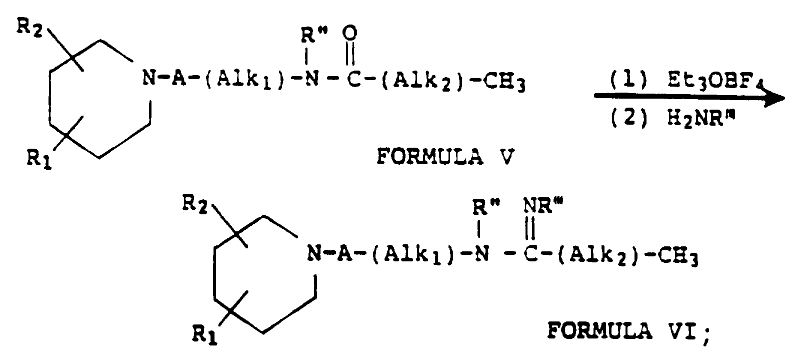 REACTION SCHEME VII
wherein compounds according to Formula VII are prepared by an amidation reaction between Intermediate I and diamine 1c, wherein Formula VII is a representation of Formula A wherein A is -CH₂- or 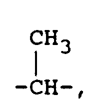 D is 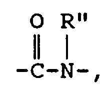 E is 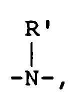 and (Alk₁), (Alk₂), (Alk₃), R", R', R₁ and R₂ are defined as above in Formula A: 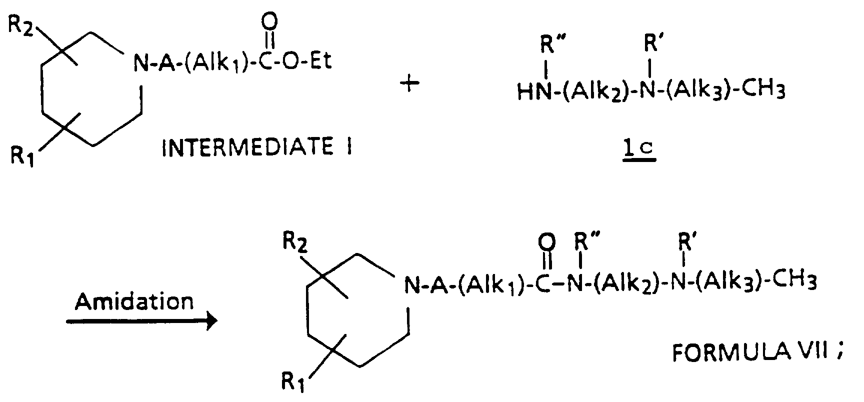 REACTION SCHEME VIII
wherein further treatment of a compound according to Formula VII forms a compound according to Formula VIII which is a representation of Formula A wherein A is -CH₂- or 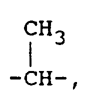 D is 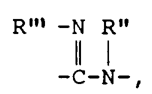 E is 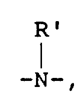 and (Alk₁), (Alk₂), (Alk₃), R', R", R''', R₁ and R₂ are defined as above in Formula A: 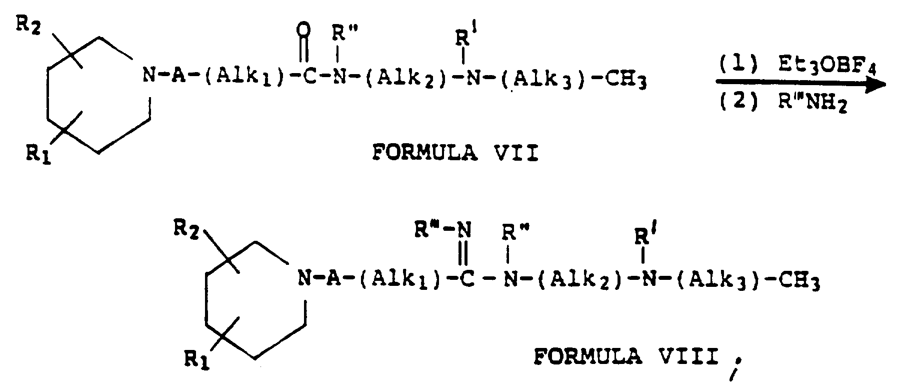 REACTION SCHEME IX
wherein compounds according to Formula IX is prepared by an amidation reaction between Intermediate III and a substituted amine 2a': 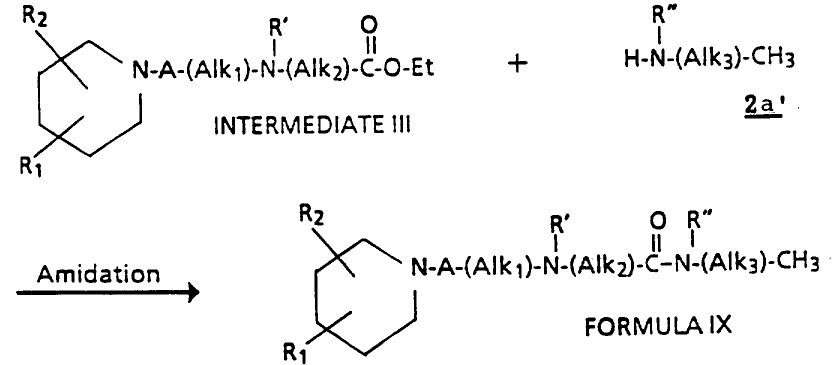 wherein Formula IX is a representation of Formula A wherein A is -CH₂- or 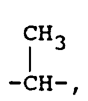 D is 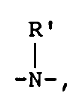 E is 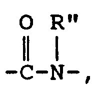 and (Alk₁), and (Alk₂), (Alk₃), R', R", R₁ and R₂ are defined as above in formula A;
REACTION SCHEME X
wherein a compound according to Formula IX is converted to the corresponding amidine according to Formula X which is a representation of Formula A wherein A is -CH₂- or 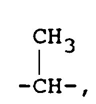 D is 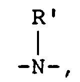 E is 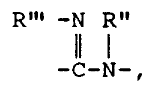 and (Alk₁), (Alk₂), (Alk₃), R', R", R''' , R₁ and R₂ are defined as above in Formula A: 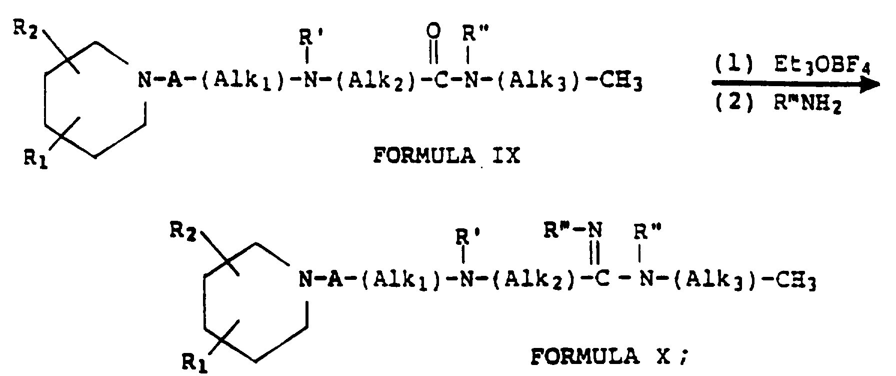 REACTION SCHEME XI
wherein compounds according to Formula XI are prepared by an amidation reaction between the alkylamine ester 3a and the Intermediate IIa', wherein Formula XI is a representation of Formula A wherein A is 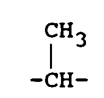 or -CH₂-, D is 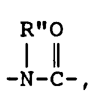 E is 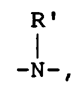 and (Alk₁), (Alk₂), (Alk₃), R', R", R₁ and R₂ are defined as above in Formula A: 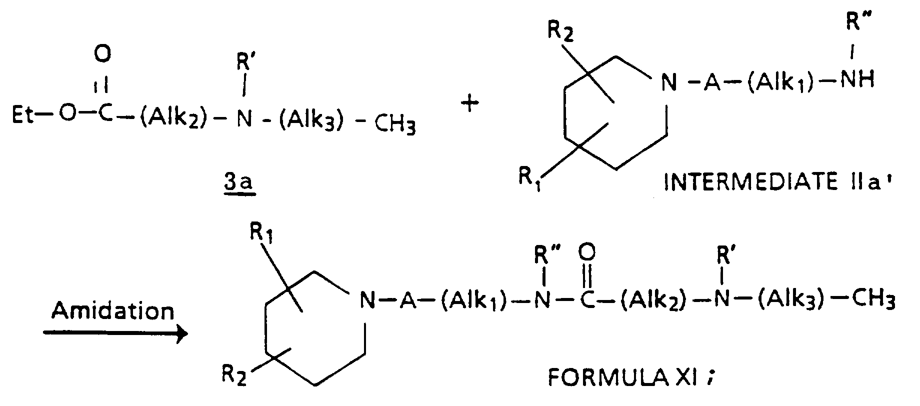 REACTION SCHEME XII
wherein an amide according to Formula XI is converted to the corresponding amidine according to Formula XII which is a representation of Formula A wherein D is 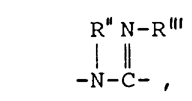 E is 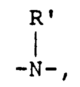 A is -CH₂- or 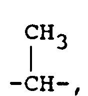 (Alk₃) is an alkylene moiety of 0 carbon atoms, and (Alk₁), (Alk₂), R₁, R₂ and R', R" and R''' are defined as in Formula A: 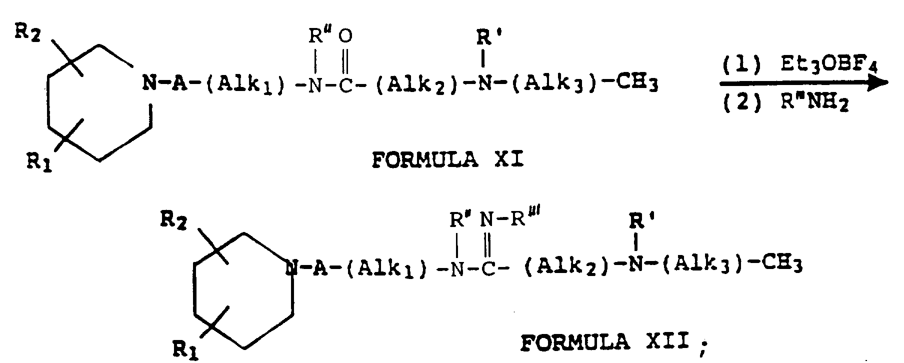 REACTION SCHEME XIII
wherein an amide according to Formula XIII is prepared by an N-alkylation of the tosylate 4d by Intermediate IIa, wherein Formula XIII is a representation of Formula A wherein A is -CH₂- or 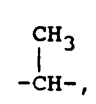 D is 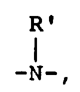 E is 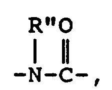 and (Alk₁), (Alk₂), (Alk₃), R', R", R₁ and R₂ are defined as above in Formula A : 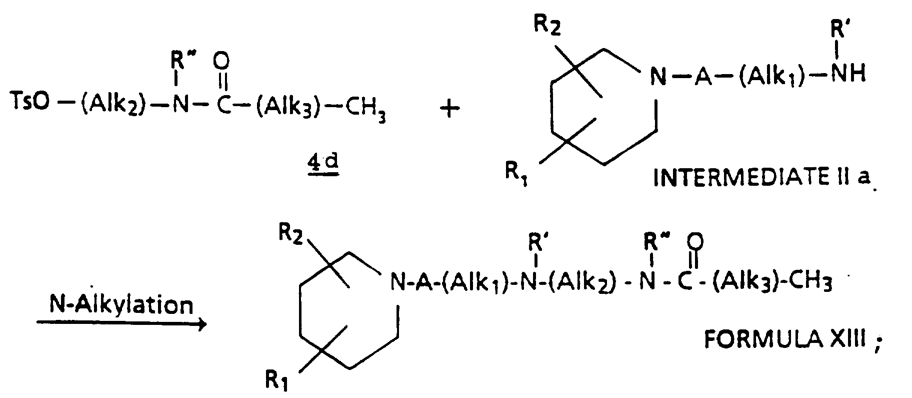 REACTION SCHEME XIV
wherein an amide according to Formula XIII is converted to an amidine according to Formula XIV which is a representation of Formula A wherein A is -CH₂- or 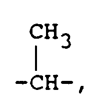 D is 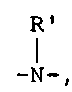 E is 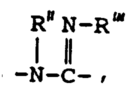 and (Alk₁) , (Alk₂), (Alk₃), R₁, R₂, R', R" and R''', are defined as above: 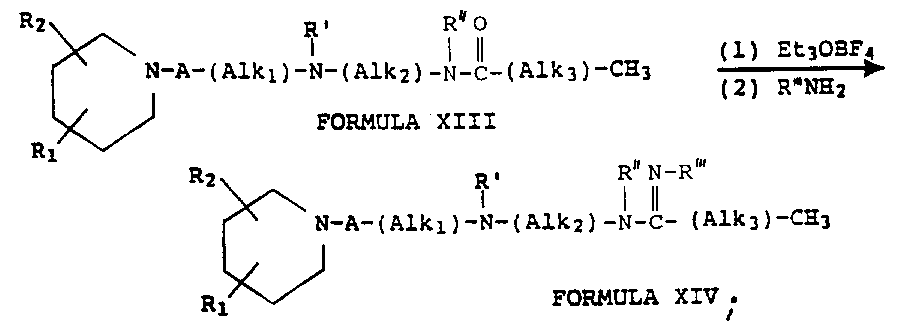 REACTION SCHEME XV
wherein compounds according to Formula XV is prepared by an amidation reaction between the imidate ester 3b', and Intermediate A wherein Formula XV is a representation of Formula A wherein A is 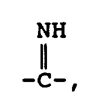 both D and E represent direct bonds and (Alk₁), (Alk₂). and (Alk₃) are represented by (Alkₓ), in which x is an integer from 1 to 18: 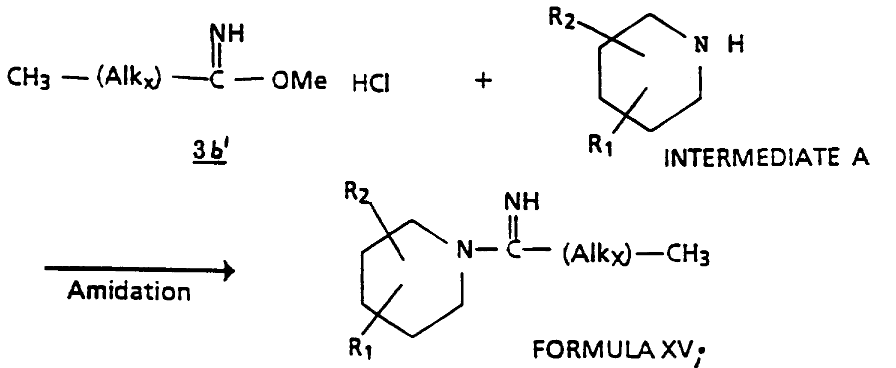 REACTION SCHEME XVI
wherein compounds according to Formula XVI is prepared by reacting Intermediate A with an aldehyde or a ketone 1e' wherein X is hydrogen or -CH₃ and wherein Formula XVI is a representation of Formula A wherein A is -CHX, wherein X is hydrogen or -CH₃, and D and E are direct bonds: 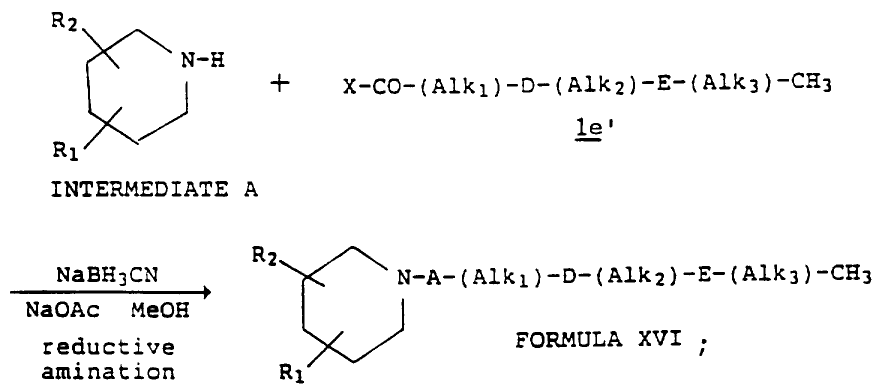 REACTION SCHEME XVII
wherein compounds according to Formula XVII is prepared by reducing a trifluoromethylalkyl substituted piperidine ester 4e wherein Formula XVII is a representation of Formula A wherein A is -CH(CF₃)-, and R₁, R₂, R', R", R", D, E, (Alk₁), (Alk₂) and (Alk₃) are all defined as above in Formula A:

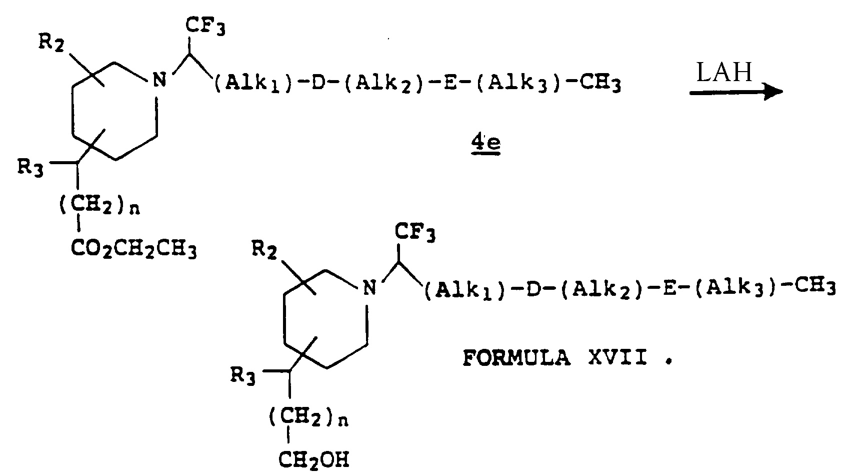

## Patentansprüche

1. Verbindung der Formel A 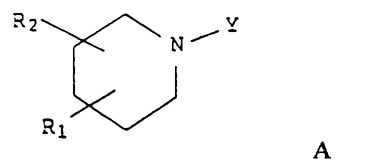 sowie deren pharmazeutisch verträgliche Salze,
wobei Y -A-(Alk₁)-D-(Alk₂)-E-(Alk₃)-CH₃ bedeutet, wobei A -CH₂-, 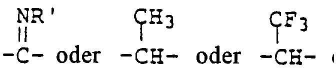 darstellt, D und E jeweils unabhängig -CH₂-, 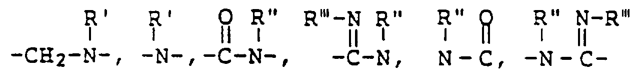 oder eine direkte Bindung bedeuten, mit der Maßgabe, daß wenn D eine Einheit aus der Gruppe 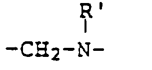 oder 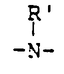 darstellt, E nicht eine Einheit aus der gleichen Gruppe sein kann, und daß wenn D eine Einheit aus der Gruppe 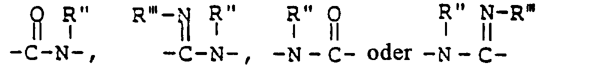 darstellt, E nicht eine Einheit aus der gleichen Gruppe sein kann; und mit der Maßgabe, daß wenn A -CH(CF₃) bedeutet, D und/oder E nicht eine Einheit aus der Gruppe 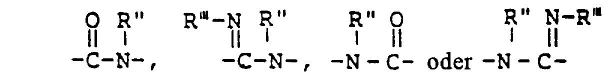 sein können;
wobei (Alk₁), (Alk₂) und (Alk₃) jeweils unabhängig eine geradkettige Alkyleneinheit mit null bis 5 Kohlenstoffatomen bedeuten, die gegebenenfalls mit 1 bis 3 Methylgruppen substituiert ist, mit der Maßgabe, daß (Alk₂) nicht den Wert null für die Kohlenstoffatome aufweisen kann; oder
(Alk₁), (Alk₂) und (Alk₃) jeweils unabhängig eine geradkettige Alkenyleneinheit mit 2 bis 6 Kohlenstoffatomen bedeuten, wobei die geradkettige Alkenyleneinheit 1 bis 2 Doppelbindungen aufiveist, und gegebenenfalls mit 1 bis 3 Methylgruppen substituiert ist;
wobei R₁ einen Rest der Formel 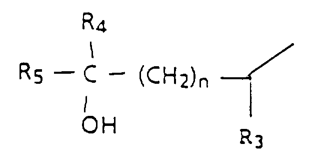 bedeutet, der sich entweder an der 3- oder der 4-Position des Piperidinrings befindet, wobei R₃ einen (C₁-C₄)-Niederalkylrest darstellt, R₄ und R₅ jeweils unabhängig ein Wasserstoffatom oder einen (C₁-C₄)-Niederalkylrest bedeuten, und n null beträgt oder eine ganze Zahl von 1 bis 3 ist;
R2 ein Wasserstoffatom oder bis zu 3 unabhängige Hydroxy- oder (C₁-C₄)-Niederalkyl-substituenten bedeutet; und
R', R" und R''' jeweils unabhängig ein Wasserstoffatom oder einen (C₁-C₄)-Niederalkylrest darstellen.

2. Verbindung nach Anspruch 1, wobei R₁ eine Gruppe der Formel 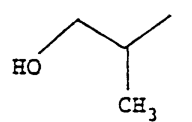 darstellt.

3. Verbindung nach Anspruch 1, die
4-(2-Hydroxy-1-methylethyl)-N-(3-methylbutyl)-1-piperidinbutanamid,
β-Methyl-1-[4-[(3-methylbutyl)amino]butyl]-4-piperidinethanol,
1-(1-Imino-4-methylpentyl)-β-methyl-4-piperidinethanol,
N-Butyl-4-(2-hydroxy-1-methylethyl)-N-methyl-1-piperidinbutanamid,
N- [4- [4-(2-Hydroxy-1-methylethyl)-1-piperidinyl]butyl]-4-methylpentanimidamid,
1-(1-Iminooctyl)-β-methyl-4-piperidinethanol,
1-(1,5,9-Trimethyldecyl)-β-methyl-4-piperidinethanol,
2-[1-(1-Trifluormethyl)undecyl-4-piperidinyl]propanol
sowie deren pharmazeutisch verträgliche Salze darstellt.

4. Verbindung, die
4-(2-Hydroxy-1-methylethyl)-1-piperidinbutannitril,
1-(4-Aminobutyl)-β-methyl-4-piperidinethanol oder 2- [(1-Phenylmethyl)-4-piperidinyliden]-1-propanol sowie deren pharmazeutisch verträgliche Salze darstellt.

5. Zusammensetzung, umfassend eine Verbindung nach einem der Ansprüche 1 bis 3, die in einem Gemisch mit einem inerten Träger vorliegt.

6. Arzneimittel umfassend eine Verbindung nach einem der Ansprüche 1 bis 3, die in einem Gemisch mit einem pharmazeutisch verträglichen Träger vorliegt.

7. Arzneimittel nach Anspruch 6 zur Senkung des Cholesterins im Plasma von Säugern.

8. Arzneimittel nach Anspruch 6 zur Behandlung von Pilzinfektionen.

9. Verwendung einer Verbindung nach einem der Ansprüche 1 bis 3 in einem Gemisch mit einem pharmazeutisch verträglichen Träger zur Herstellung eines Arzneimittels.

10. Verwendung nach Anspruch 9, wobei das Arzneimittel zur Senkung der Cholesteringehalte im Plasma wirksam ist.

11. Verwendung nach Anspruch 9, wobei das Arzneimittel bei der Behandlung von Pilzinfektionen wirksam ist.

12. Verfahren zur Herstellung eines Arzneimittels nach den Ansprüchen 6 bis 8, umfassend Kombinieren einer Verbindung nach einem der Ansprüche 1 bis 3 mit einem pharmazeutisch verträglichen Träger.

13. Verfahren zur Herstellung einer Verbindung nach einem der Ansprüche 1 bis 3, dadurch gekennzeichnet, daß das Verfahren wenigstens eine Umsetzung nach einem der folgenden Reaktionsschemata umfaßt:
Reaktionsschema I
wobei Verbindungen der Formel I, die ein Vertreter der Formel A sind, wobei D 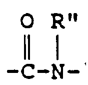 bedeutet, A -CH₂- oder 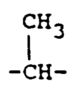 ist,
(Alk₃) eine Alkyleneinheit mit null Kohlenstoffatomen darstellt, (Alk₁) und (Alk₂) sowie R" wie in Formel A definiert sind, und E eine direkte Bindung ist,
durch eine Amidierung zwischen Zwischenprodukt I und einem substituierten Amin 2a hergestellt werden: 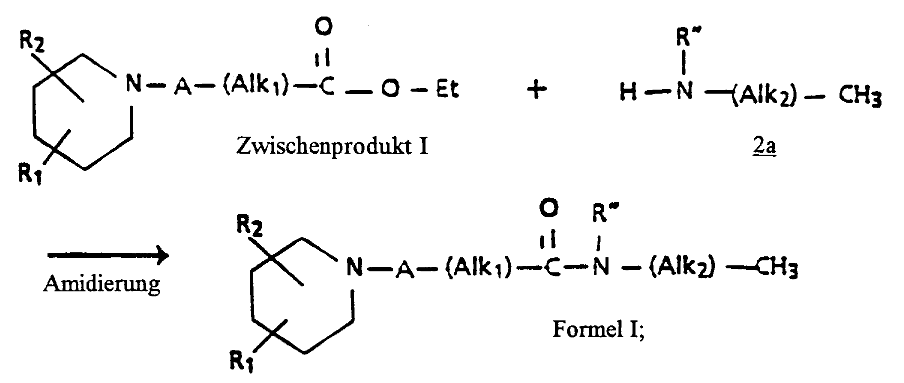
Reaktionsschema II
wobei ein Amid der Formel I zum entsurechenden Amin der Formel II,
die ein Vertreter der Formel A ist, wobei D 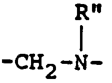 ist, A -CH₂- oder 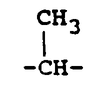 ist, E eine direkte Bindung bedeutet, (Alk₃) eine Alkyleneinheit mit null Kohlenstoffatomen darstellt, und (Alk₁), (Alk₂), R₁, R₂ sowie R" wie in Formel I definiert sind, reduziert wird: 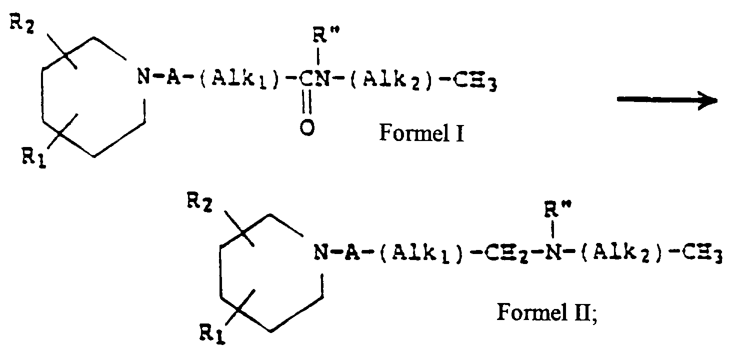
Reaktionsschema III
wobei das Amid der Formel I in das entsprechende Amidin der Formel III,
die ein Vertreter der Formel A ist, wobei D 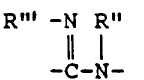 ist, A -CH₂- oder 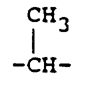 darstellt, (Alk₃) eine Alkyleneinheit mit null Kohlenstoffatomen bedeutet, E eine direkte Bindung bedeutet, und (Alk₁), (Alk₂), R₁, R₂, R" sowie R''' wie in Formel A definiert sind,
umgewandelt wird: 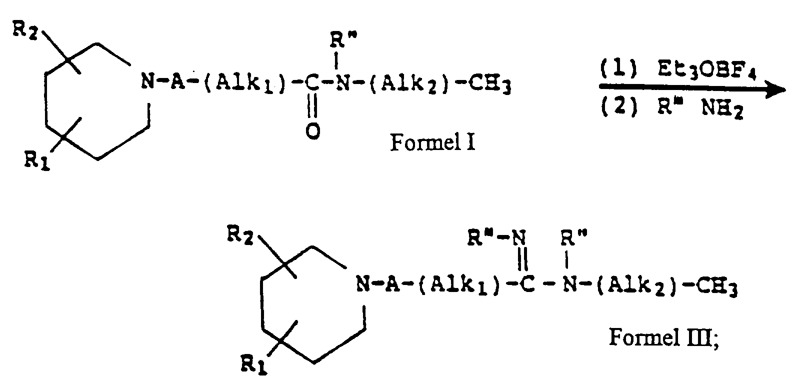
Reaktionsschema IV
wobei Anidine der Formel IV,
die ein Vertreter der Formel A ist, wobei D 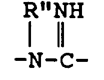 bedeutet, R" ein Wasserstoffatom bedeutet, A -CH₂- oder 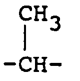 ist, (Alk₃) eine Alkyleneinheit mit null Kohlenstoffatomen darstellt, E eine direkte Bindung ist, und (Alk₁), (Alk₂), R₁ sowie R₂ wie in Formel A definiert sind,
durch eine Amidinbildungsreaktion zwischen Zwischenprodukt IIb und dem Imidatester 3b hergestellt werden: 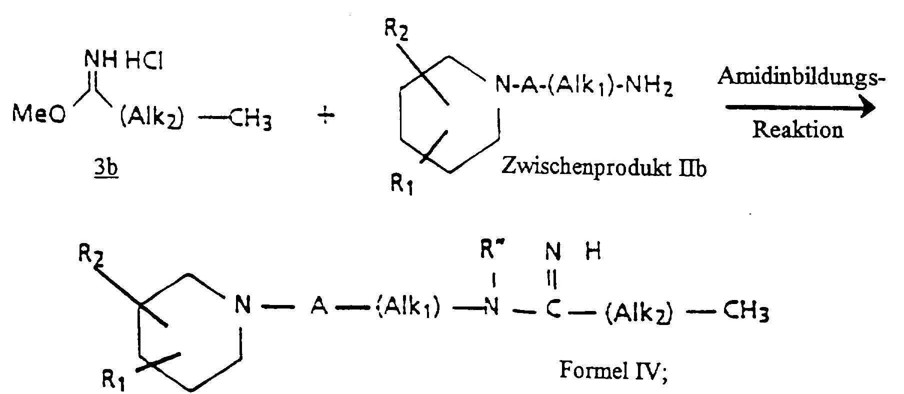 Reaktionsschema V
wobei das Amidin der Formel IV hydrolysiert wird, um ein Amid der Formel V, die ein Vertreter der Formel A ist, wobei D 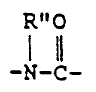 ist, A -CH₂- oder 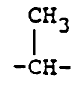 bedeutet, (Alk₃) eine Alkyleneinheit mit null Kohlenstoffatomen darstellt, E eine direkte Bindung bedeutet, und (Alk₁), (Alk₂), R₁, R₂ sowie R" wie in Formel A definiert sind, zu erzeugen: 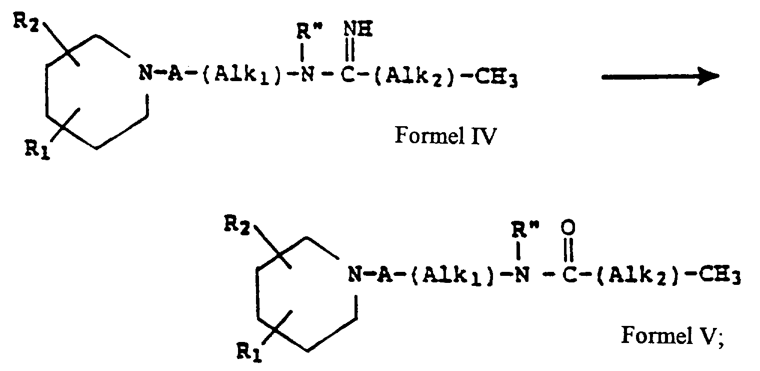 Reaktionsschema VI
wobei das Amid der Formel V in ein substituiertes Amidin der Formel VI, die ein Vertreter der Formel A ist, wobei D 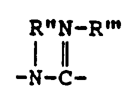 ist, A -CH₂- oder 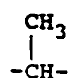 ist, (Alk₃) eine Alkyleneinheit mit null Kohlenstoffatomen bedeutet, E eine direkte Bindung ist, und (Alk₁), (Alk₂), R₁, R₂, R" sowie R''' wie in Formel A definiert sind, umgewandelt wird: 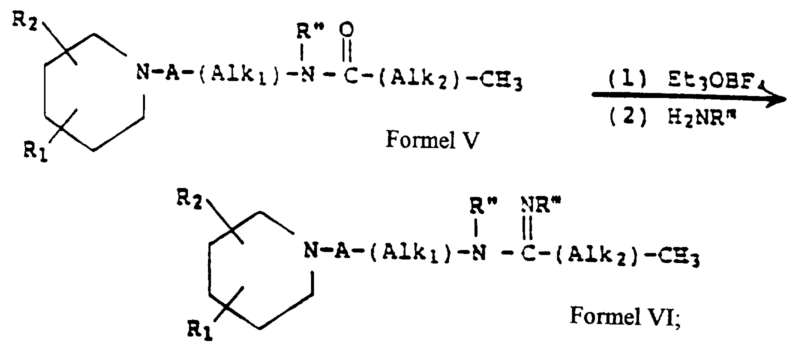 Reaktionsschema VII
wobei Verbindungen der Formel VII durch eine Amidierung zwischen Zwischenprodukt I und Diamin 1c hergestellt werden, wobei Formel VII ein Vertreter der Formel A ist, wobei A -CH₂- oder 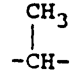 darstellt, 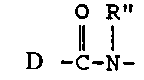 ist, E 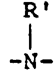 bedeutet, und (Alk₁), (Alk₂), (Alk₃), R", R', R₁ sowie R₂ wie vorstehend in Formel A definiert sind: 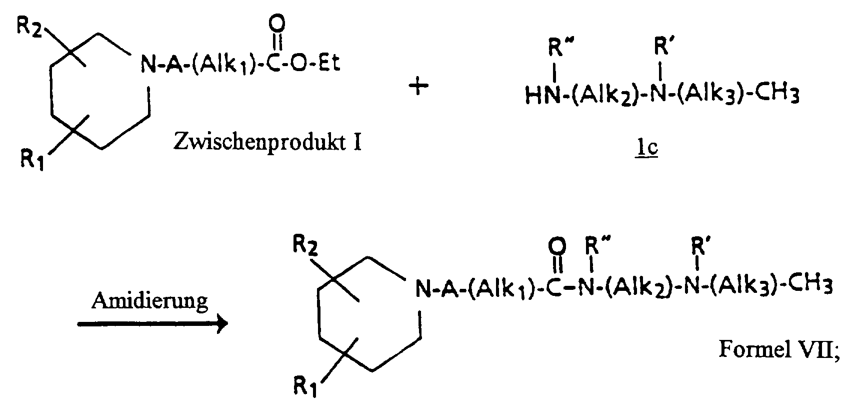 Reaktionsschema VIII
wobei durch eine weitere Behandlung einer Verbindung der Formel VII eine Verbindung der Formel VIII,
die ein Vertreter der Formel A ist, wobei A -CH₂- oder 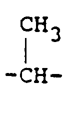 ist, D 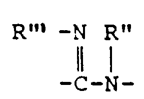 darstellt, E 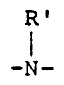 bedeutet, und (Alk₁), (Alk₂), (Alk₃), R', R", R"', R₁ sowie R₂ wie vorstehend in Formel A definiert sind,
erzeugt wird: 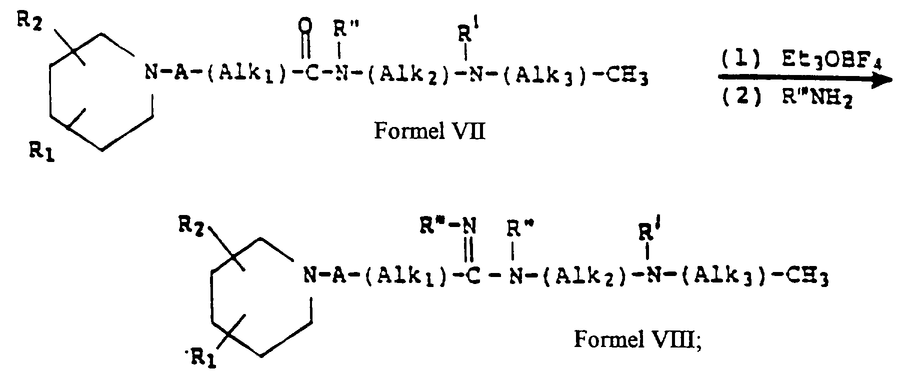 Reaktionsschema IX
wobei Verbindungen der Formel IX durch eine Amidierung zwischen Zwischenprodukt III und einem substituierten Amin 2a' hergestellt werden: 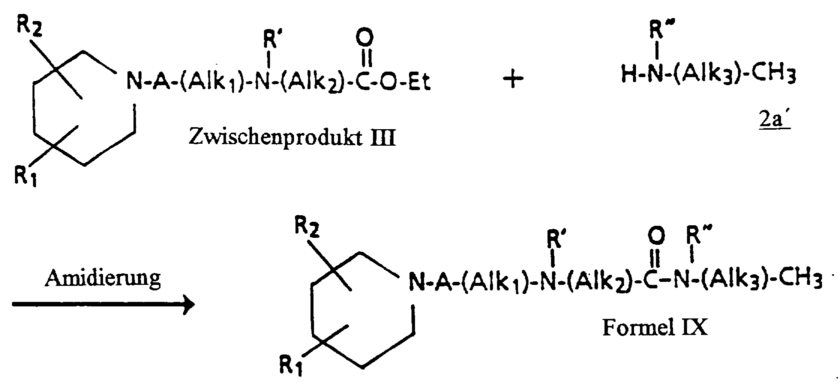 wobei Formel IX ein Vertreter der Formel A ist, wobei A -CH₂- oder 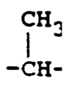 ist, D 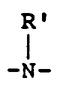 darstellt, E 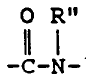 bedeutet, und (Alk₁), (Alk₂), (Alk₃), R', R", R₁ sowie R₂ wie vorstehend in Formel A definiert sind;
Reaktionsschema X
wobei eine Verbindung der Formel IX in das entsprechende Amidin der Formel X, die ein Vertreter der Formel A ist, wobei A -CH₂- oder 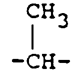 ist, D 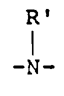 darstellt, E 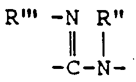 bedeutet, und (Alk₁), (Alk₂), (Alk₃), R', R", R"', R₁ sowie R₂ wie vorstehend in Formel A definiert sind,
umgewandelt wird: 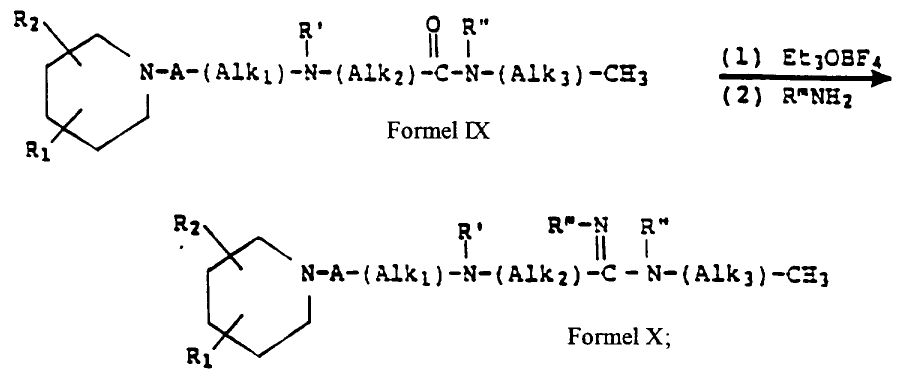 Reaktionsschema XI
wobei Verbindungen der Formel XI durch eine Amidierung zwischen einem Alkylaminester 3a und dem Zwischenprodukt IIa' hergestellt werden, wobei Formel XI ein Vertreter der Formel A ist, wobei A 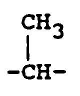 oder -CH₂- darstellt, 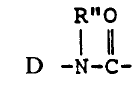 ist, E 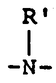 bedeutet, und (Alk₁), (Alk₂), (Alk₃), R', R", R₁ sowie R₂ wie vorstehend in Formel A definiert sind: 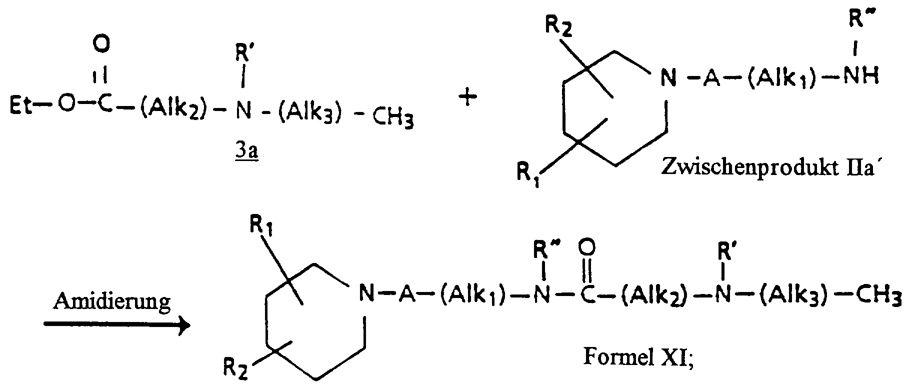 Reaktionsschema XII
wobei ein Amid der Formel XI in das entsprechende Amidin der Formel XII, die ein Vertreter der Formel A ist, wobei D 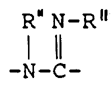 darstellt, E 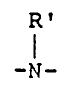 bedeutet, A -CH₂- oder 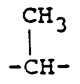 ist, (Alk₃) eine Alkyleneinheit mit null Kohlenstoffatomen bedeutet, und (Alk₁), (Alk₂), R₁, R₂, R', R" sowie R''' wie in Formel A definiert sind, umgewandelt wird: 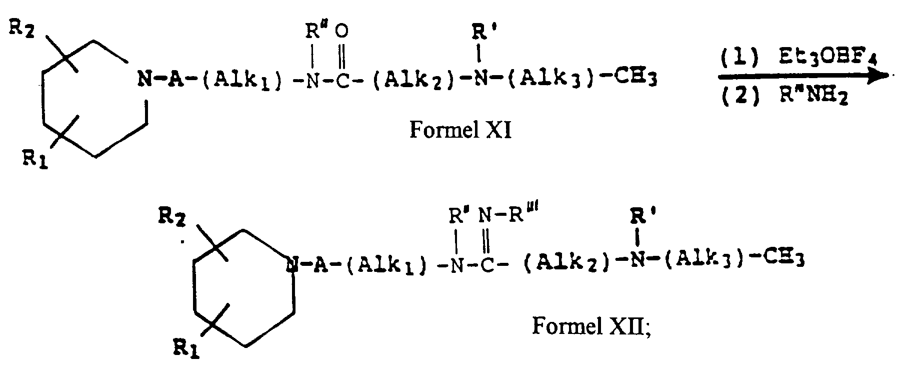 Reaktionsschema XIII
wobei ein Amid der Formel XIII durch eine N-Alkylierung des Tosylats 4d durch das Zwischenprodukt IIa hergestellt wird, wobei Formel XIII ein Vertreter der Formel A ist, wobei A -CH₂- oder 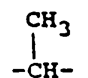 ist, D 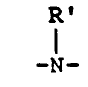 Darstellt, E 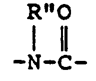 bedeutet, und (Alk₁), (Alk₂), (Alk3), R', R", R₁ sowie R₂ wie vorstehend in Formel A definiert sind: 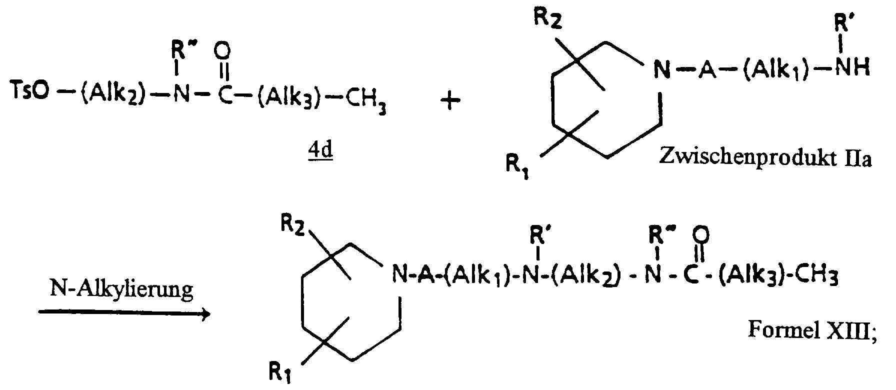 Reaktionsschema XIV
wobei ein Amid der Formel XIII in das entsprechende Amidin der Formel XIV, die ein Vertreter der Formel A ist, wobei A -CH₂- oder 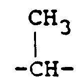 ist, D 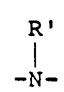 darstellt, E 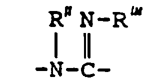 bedeutet, und (Alk₁), (Alk₂), (Alk₃), R₁, R₂, R', R" sowie R''' wie vorstehend in Formel A definiert sind,
umgewandelt wird: 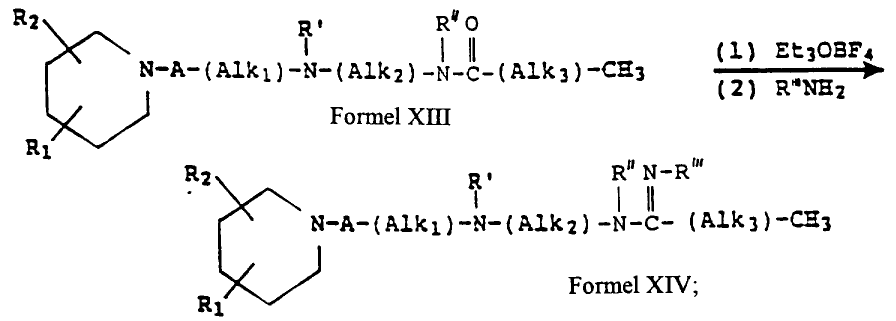 Reaktionsschema XV
wobei Verbindungen der Formel XV durch eine Amidierung zwischen dem Imidatester 3b' und dem Zwischenprodukt A hergestellt werden, wobei Formel XV ein Vertreter der Formel A ist, wobei A 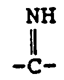 darstellt, sowohl D als auch E direkte Bindungen bedeuten, und (Alk₁), (Alk₂) sowie (Alk₃) durch (Alkₓ) wiedergeben werden, wobei x eine ganze Zahl von 1 bis 18 ist: 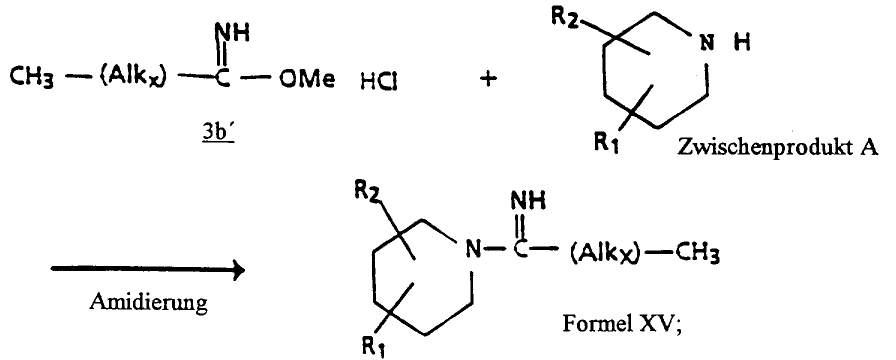 Reaktionsschema XVI
wobei Verbindungen der Formel XVI durch Umsetzen des Zwischenprodukts A mit einem Aldehyd oder einem Keton 1e' hergestellt werden,
wobei X ein Wasserstoffatom oder eine -CH₃-Gruppe ist, und wobei Formel XVI ein Vertreter der Formel A ist, wobei A -CHX darstellt, wobei X ein Wasserstoffatom oder eine -CH3-Gruppe ist, und D sowie E direkte Bindungen bedeuten: 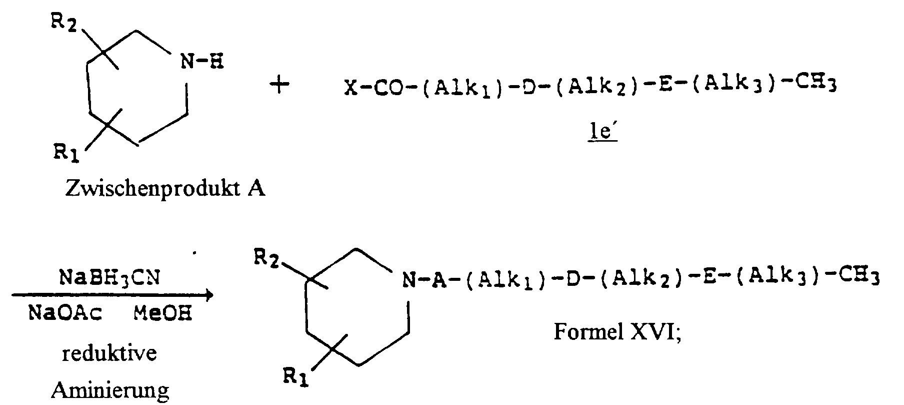
Reaktionsschema XVII
wobei Verbindungen der Formel XVII durch Reduzieren eines trifluormethylalkylsubstituierten Piperidinesters 4e hergestellt werden,
wobei Formel XVII ein Vertreter der Formel A ist, wobei A -CH(CF₃)- darstellt, und R₁, R₂, R', R", R''', D, E, (Alk₁), (Alk₂) sowie (Alk₃) alle wie vorstehend in Formel A definiert sind:

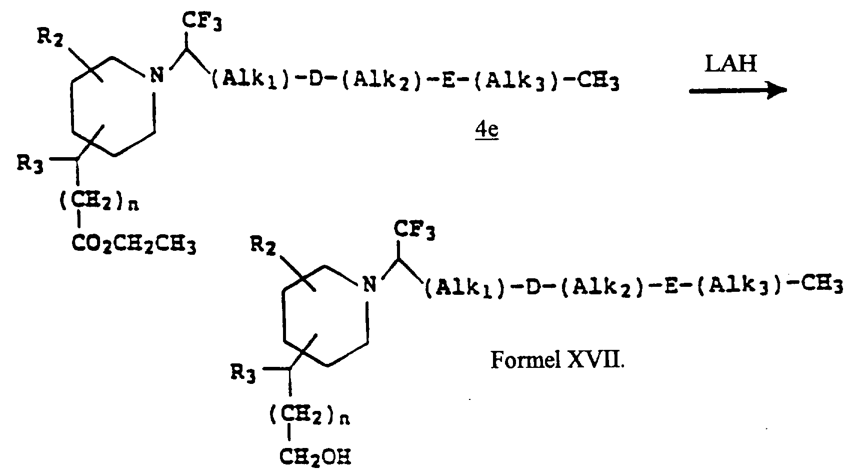

## Revendications

1. Composé répondant à la formule A : 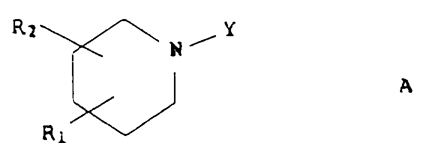 et ses sels acceptables du point de vue pharmaceutique, dans lesquels Y représente -A-(Alk₁)-D-(Alk₂)-E-(Alk₃)-CH₃ dans lequel A représente 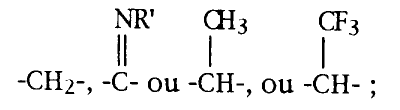 D et E représentent chacun indépendamment -CH₂-, 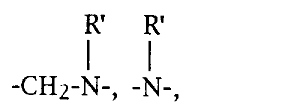 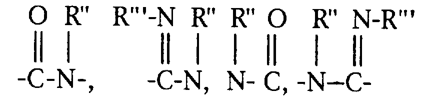 ou une liaison directe, à la condition que lorsque D représente un fragment du groupe 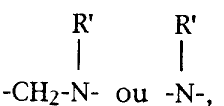 E ne puisse représenter un fragment du même groupe, et que lorsque D représente un fragment du groupe 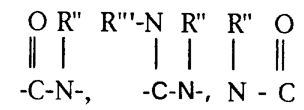 ou 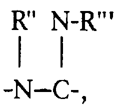 E ne puisse représenter un fragment du même groupe ; et à la condition que lorsque A représente -CH(CF₃), D et/ou E ne puissent représenter un fragment du groupe 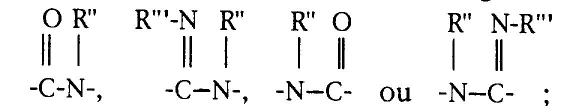 (Alk₁), (Alk₂) et (Alk₃) représentent chacun indépendamment un fragment akylène à chaîne linéaire contenant de 0 à 5 atomes de carbone, éventuellement substitué par 1 à 3 groupes méthyle, à la condition que (Alkz) ne puisse comprendre 0 atomes de carbone ; ou bien (Alk₁), (Alk₂) et (Alk₃) représentent chacun indépendamment un fragment alcénylène à chaîne linéaire contenant de 2 à 6 atomes de carbone, le fragment alcénylène comportant de 1 à 2 double liaisons, et étant éventuellement substitué par 1 à 3 groupes méthyle ;
R₁ représente un groupe de formule 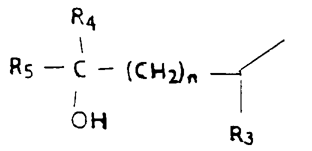 situé en position 3 ou en position 4 du cycle pipéridine, dans lequel
R₃ représente un groupe alkyle inférieur en C₁₋₄, R₄ et R₅ représentent chacun indépendamment un atome d'hydrogène ou un groupe alkyle inférieur en C₁₋₄ et n est égal à 0 ou à un nombre entier compris entre 1 et 3 ;
R₂ représente un atome d'hydrogène ou jusqu'à 3 substitutions indépendantes hydroxy ou alkyle inférieur en C₁₋₄; et R', R" et R"' représentent chacun indépendamment un atome d'hydrogène ou un groupe alkyle inférieur en C₁₋₄.

2. Composé selon la revendication 1 dans lequel R₁ représente un groupe de formule

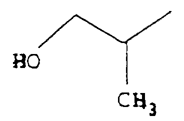

3. Composé selon la revendication 1 qui est le 4-(2-hydroxy-1-méthyléthyl)-N-(3-méthylbutyl)-1-pipéridinebutanamide, le β-méthyl-1-[4-[3-méthylbutyl)-amino]butyl-4-pipéridlneéthanol, le 1-(1-imino-4-méthylpentyl)-β-méthyl-4-pipéridineéthanol, le N-butyl-4-(2-hydroxy-1-méthyléthyl )-N-méthyl-1-pipéridinebutanamide, le N-[4-[4-(2-hydroxy-1-méthyléthyl)-1-pipéridinyl]butyl]-4-méthylpentanimidamide, le 1-(1-hninooctyl)-β-méthyl-4-pipéridineéthanol, le 1-(1,5,9-triméthyldécyl)-β-méthyl-4-piperidineéthanol ou le 2-[1-(1-trifluorométhyl)undécyl-4-pipéridinyl]propanol, et leurs sels acceptables du point de vue pharmaceutique.

4. Composé qui est le 4-(2-hydroxy-1-méthyléthyl)-1-pipéridinebutanenitrile, le 1-(4-aminobutyl)-β-méthyl-4-pipéridineéthanol ou le 2-(1-phénylméthyl) -4-pipéridinylidène]-1-propanol, et leurs sels acceptables du point de vue pharmaceutique.

5. Composition comprenant un composé selon l'une quelconque des revendications 1 à 3 présent en mélange avec un véhicule inerte.

6. Composition pharmaceutique comprenant un composé de l'une quelconque des revendications 1 à 3 présent en mélange avec un véhicule acceptable du point de vue pharmaceutique.

7. Composition pharmaceutique selon la revendication 6 pour abaisser la teneur en cholestérol dans le plasma chez les mammifères.

8. Composition pharmaceutique selon la revendication 6 pour traiter les infections fongiques.

9. Utilisation d'un composé selon l'une quelconque des revendications 1 à 3 en mélange avec un véhicule acceptable du point de vue pharmaceutique, pour la préparation d'une composition pharmaceutique.

10. Utilisation selon la revendication 9, dans laquelle la composition pharmaceutique est efficace pour abaisser les teneurs en cholestérol dans le plasma.

11. Utilisation selon la revendication 9, dans laquelle la composition pharmaceutique est efficace pour traiter les infections fongiques.

12. Procédé de préparation d'une composition pharmaceutique telle que définie dans les revendications 6 à 8, comprenant la combinaison d'un composé tel que défini dans l'une quelconque des revendications 1 à 3 avec un véhicule acceptable du point de vue pharmaceutique.

13. Procédé de préparation d'un composé tel que défini dans les revendications 1 à 3, caractérisé en ce que le procédé comprend au moins une réaction conforme aux schémas de réaction suivants :
SCHEMA DE REACTION I
dans lequel des composés répondant à la formule I qui est une représentation de la formule A dans laquelle D représente 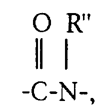 A représente -CH₂- ou 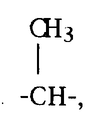 (Alk₃) représente un fragment alkylène de 0 atomes de carbone, (Alk₁) et (Alk₂) et R" sont définis comme dans la formule A, et E est une liaison directe, sont préparés par une réaction d'amidation entre l'intermédiaire I et une amine substitué 2a: 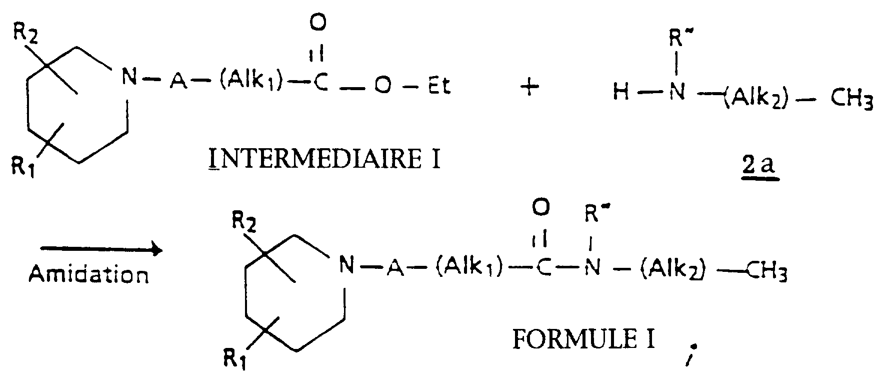 SCHEMA DE REACTION II
dans lequel un amide répondant à la formule I est réduit en l'amine correspondante de formule II qui est une représentation de la formule A dans laquelle D représente 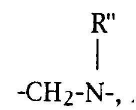 A représente -CH₂- ou 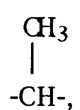 E représente une liaison directe. (Alk₃) représente un fragment alkylène de 0 atomes de carbone, et (Alk₁), (Alk₂), R₁, R₂ et R" sont définis comme dans la formule I : 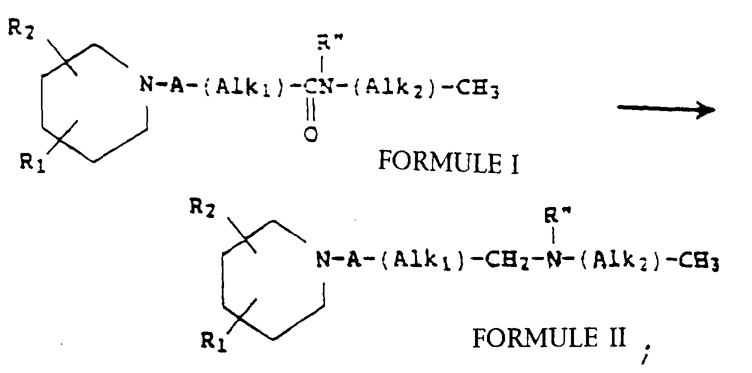 SCHEMA DE REACTION III
dans lequel l'amide répondant à la formule I est converti en l'amidine répondant à la formule III qui est une représentation de la formule A dans laquelle D représente 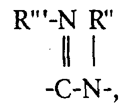 A représente -CH₂- ou 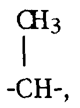 (Alk₃) représente un fragment alkylène de 0 atomes de carbone, E représente une liaison directe et (Alk₁), (Alk₂), R₁, R₂, R" et R"' sont définis comme dans la formule A : 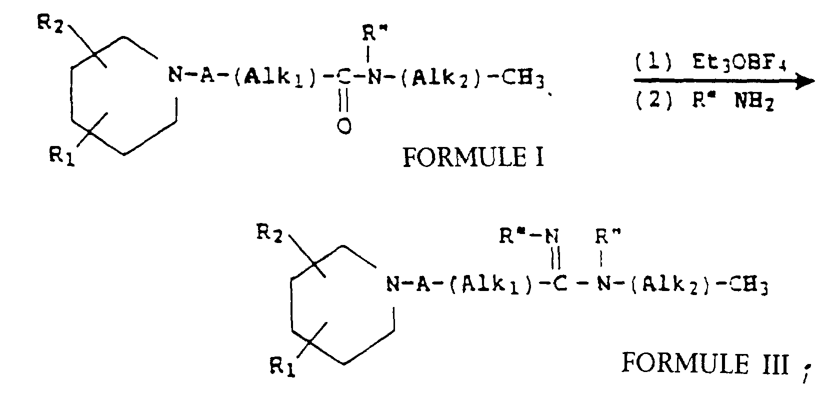 SCHEMA DE REACTION IV
dans lequel des amidines répondant à la formule IV qui est une représentation de la formule A dans laquelle D représente 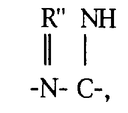 R" représente un atome d'hydrogène, A représente -CH₂- ou 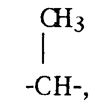 (Alk₃) est un fragment alkylène de 0 atomes de carbone, E représente une liaison directe et (Alk₁), (Alk₂), R₁ et R₂ sont définis comme dans la formule A, sont préparées par une réaction de formation d'amidine entre l'intermédiaire IIb et un ester d'imidate substitué 3b : 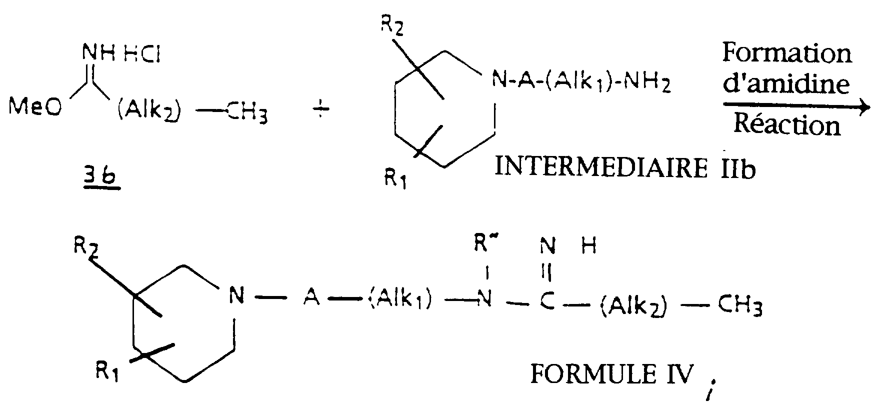 SCHEMA DE REACTION V
dans lequel l'amidine répondant à la formule IV est hydrolysée pour former un amide répondant à la formule V qui est une représentation de la formule A dans laquelle D représente 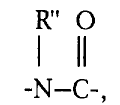 A représente -CH₂- ou 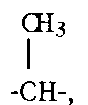 (Alk₃) représente un fragment alkylène de 0 atomes de carbone, E représente une liaison directe, (Alk₁) et (Alk₂) R₁, R₂ et R" sont définis comme dans la formule A. 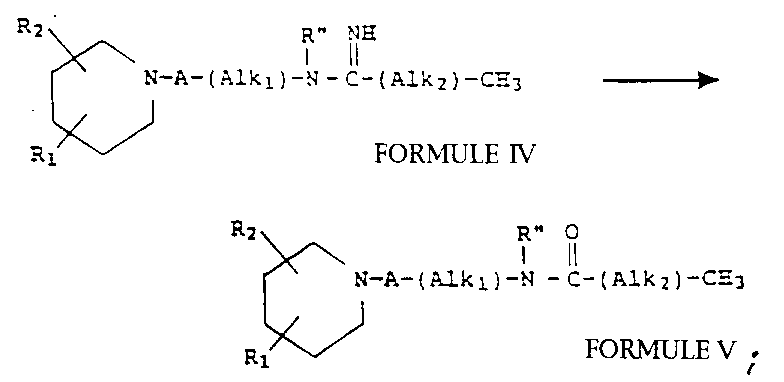 SCHEMA DE REACTION VI
dans lequel l'amidine répondant à la formule V est convertie en une amidine substituée répondant à la formule VI qui est une représentation de la formule A dans laquelle D représente 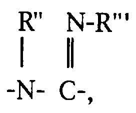 A représente -CH₂- ou 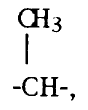 (Alk₃) représente un fragment alkylène de 0 atomes de carbone, E représente une liaison directe, (Alk₁) et (Alk₂) R₁, R₂, R" et R"' sont définis comme dans la formule A. 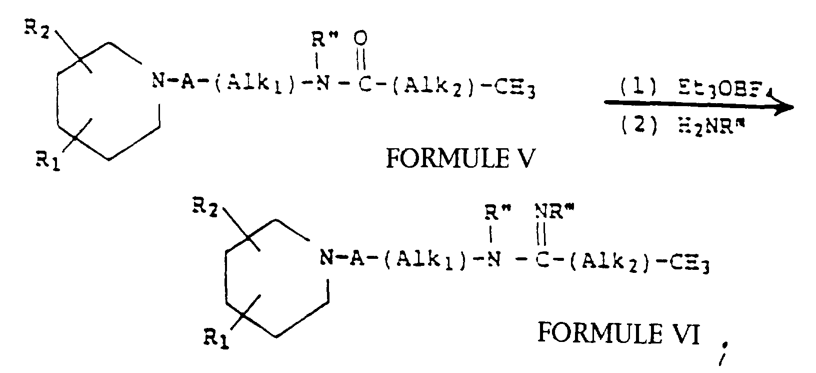 SCHEMA DE REACTION VII
dans lequel des composés répondant à la formule VII sont préparés par une réaction d'amidation entre l'intermédiaire I et la diamine 1c, la formule VII étant une représentation de la formule A dans laquelle A représente -CH2- ou 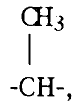 D représente 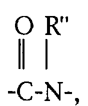 E représente 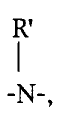 et (Alk₁), (Alk₂), (Alk₃), R", R', R₁ et R₂ sont définis comme ci-dessus dans la formule A : 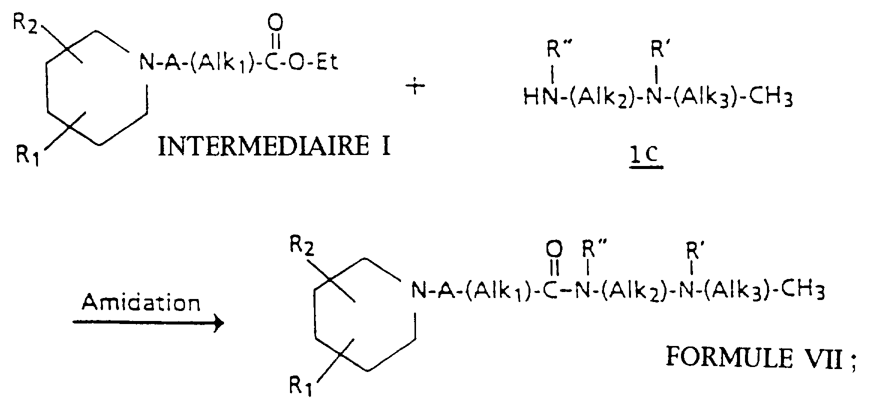 SCHEMA DE REACTION VIII
dans lequel un autre traitement d'un composé répondant à la formule VII forme un composé répondant à la formule VIII qui est une représentation de la formule A dans laauelle A représente -CH₂- ou 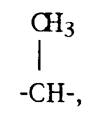 D représente 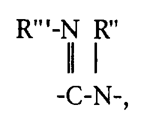 E représente 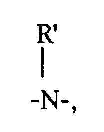 et (Alk₁), (Alk₂), (Alk₃), R', R", R"', R₁ et R₂ sont définis comme ci-dessus dans la formule A: 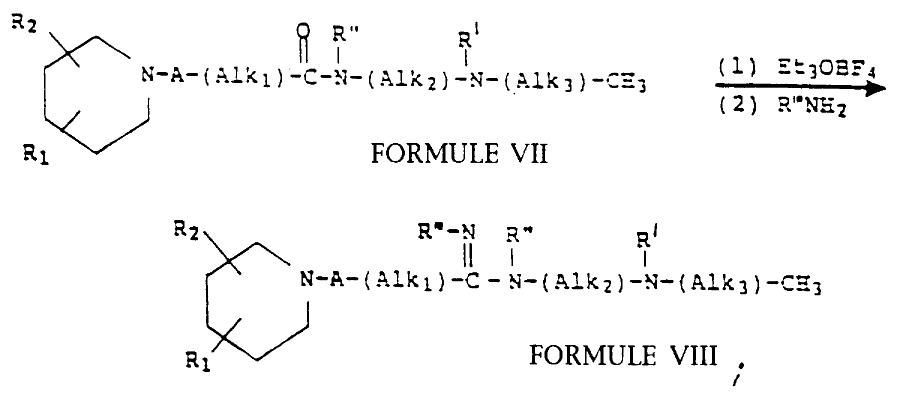 SCHEMA DE REACTION IX
dans lequel des composés répondant à la formule IX sont préparés par une réaction d'amidation entre l'intermédiaire III et une amine substituée 2a': 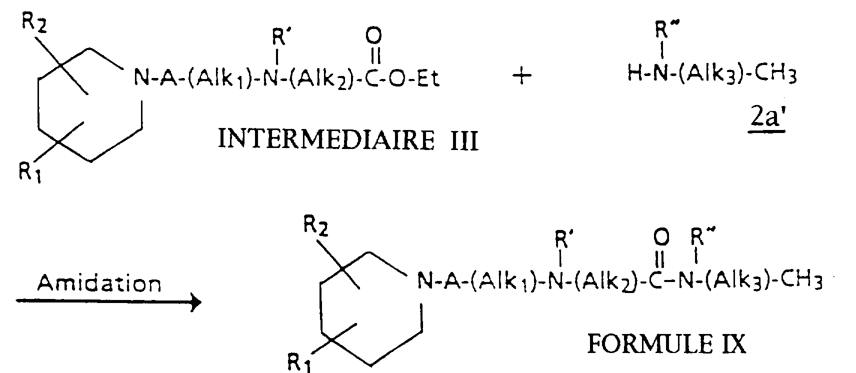 dans laquelle la formule IX est une représentation de la formule A dans laquelle A représente -CH₂- ou 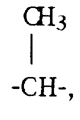 D représente 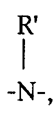 E représente 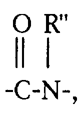 et (Alk₁), (Alk₂), (Alk₃), R', R", R₁ et R₂ sont définis comme ci-dessus dans la formule A. SCHEMA DE REACTION X
dans lequel un composé répondant à la formule IX est converti en l'amidine correspondante répondant à la formule X qui est une représentation de la formule A dans laquelle A représente -CH₂- ou 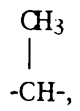 D représente 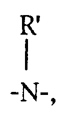 E représente 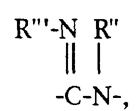 et (Alk₁), (Alk₂), (Alk₃), R', R", R"', R₁ et R₂ sont définis comme ci-dessus dans la formule A : 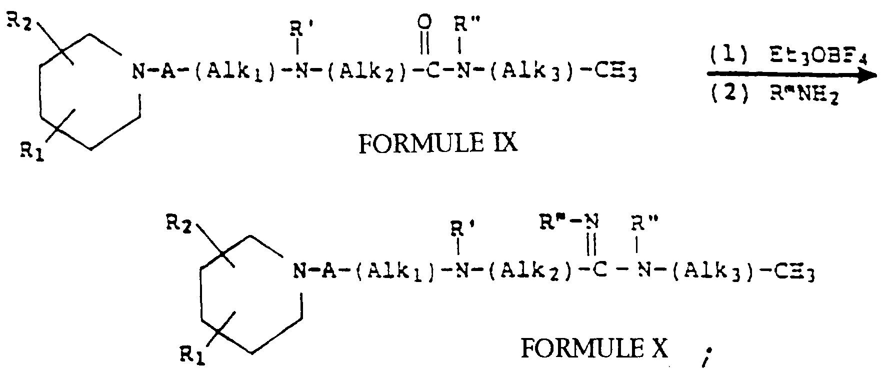 SCHEMA DE REACTION XI
dans lequel des composés répondant à la formule XI sont préparés par une réaction d'amidation entre l'ester d'alkylamine 3a et l'intermédiaire IIa', la formule XI étant une représentation de la formule A dans laquelle A représente 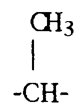 ou-CH₂-, D représente 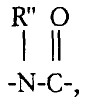 E représente 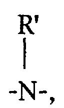 et (Alk₁), (Alk₂), (Alk₃), R', R", R₁ et R₂ et R" sont définis comme ci-dessus dans la formule A: 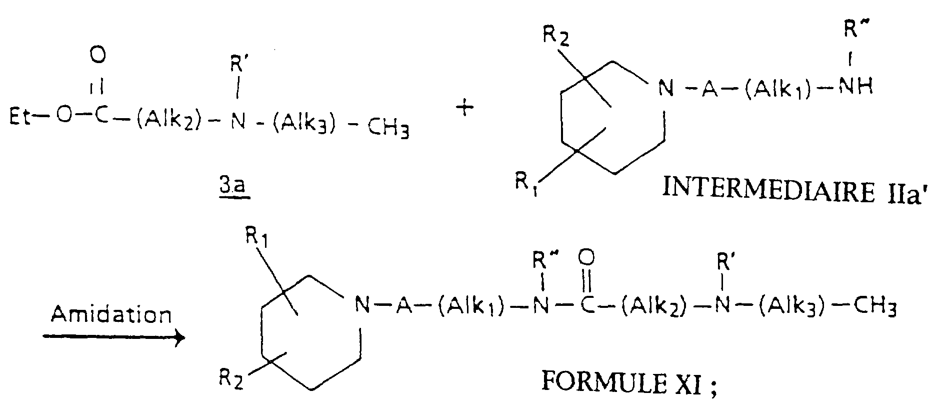 SCHEMA DE REACTION XII
dans lequel un amide répondant à la formule XI est converti en l'amidine correspondant répondant à la formule XII qui est une représentation de la formule A dans laquelle D représente 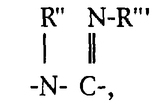 E représente 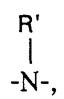 A représente -CH₂- ou 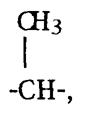 (Alk₃) est un fragment alkylène de 0 atomes de carbone, et (Alk₁), (Alk₂), R₁, R₂, R', R" et R"' sont définis comme dans la formule A: 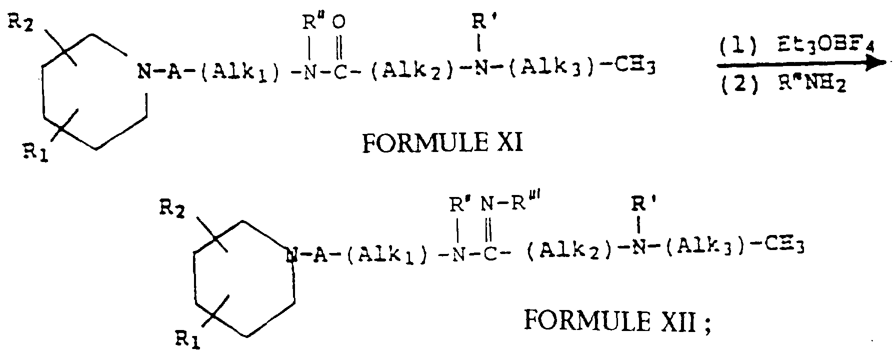 SCHEMA DE REACTION XIII
dans lequel un amide répondant à la formule XIII est préparé par une N-alkylation du tosylate 4d par l'intermédiaire IIa, la formule XIII étant une représentation de la formule A dans laquelle A représente -CH2- ou 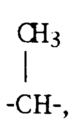 D représente 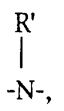 E représente 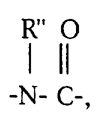 et (Alk₁), (Alk₂), (Alk₃), R', R", R₁ et R₂ sont définis comme ci-dessus dans la formule A : 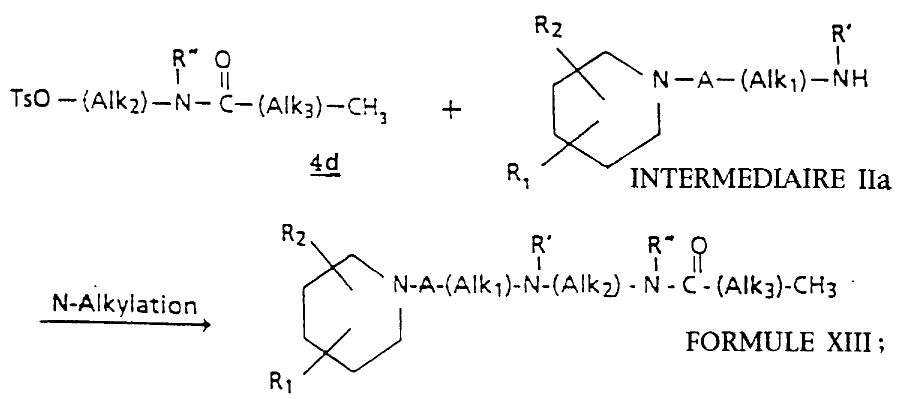 SCHEMA DE REACTION XN
dans lequel un amide répondant à la formule XIII est converti en une amidine répondant à la formule XIV qui est une représentation de la formule A dans laquelle A représente -CH₂- ou 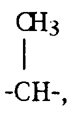 D représente 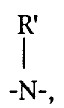 E représente 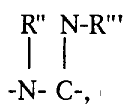 et (Alk₁), (Alk₂), (Alk₃), R₁, R₂, R', R" et R"' sont définis comme ci-dessus : 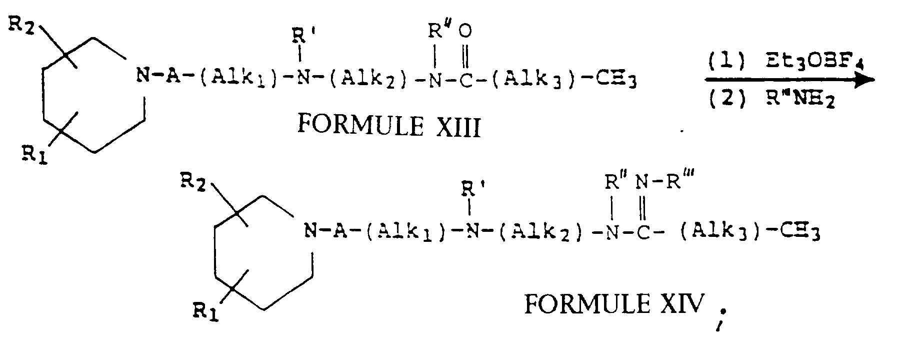 SCHEMA DE REACTION XV
dans lequel des composés répondant à la formule XV sont préparés par une réaction d'amidation entre l'ester d'imidate 3b' et l'intermédiaire A, la formule XV étant une représentation de la formule A dans laquelle A représente 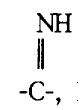 D et E représentent tous deux des liaisons directes et (Alk₁), (Alk₂), (Alk₃) sont représentés par (Alkₓ), dans lequel x est un nombre entier compris entre 1 et 18 : 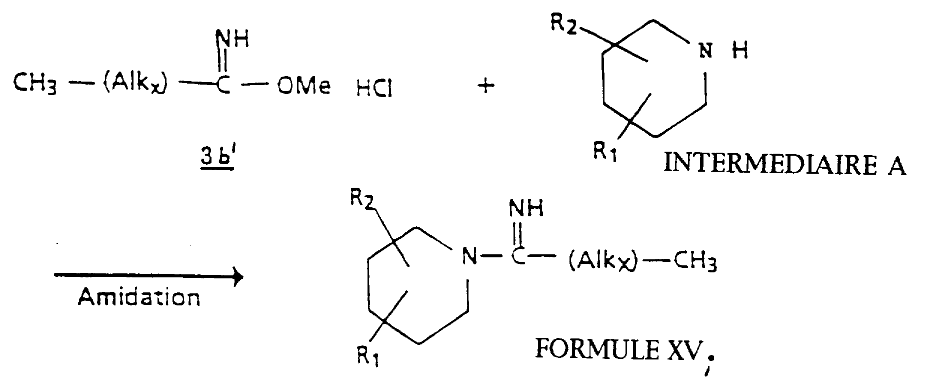 SCHEMA DE REACTION XVI
dans lequel des composés répondant à la formule XVI sont préparés en faisant réagir un intermédiaire A avec un aldéhyde ou une cétone 1e', dans lesquels X représente un atome d'hydrogène ou -CH₃, la formule XVI étant une représentation de la formule A, dans laquel A représente -CHX, dans lequel X représente un atome d'hydrogène ou -CH₃, et D et E représentent des liaisons directes : 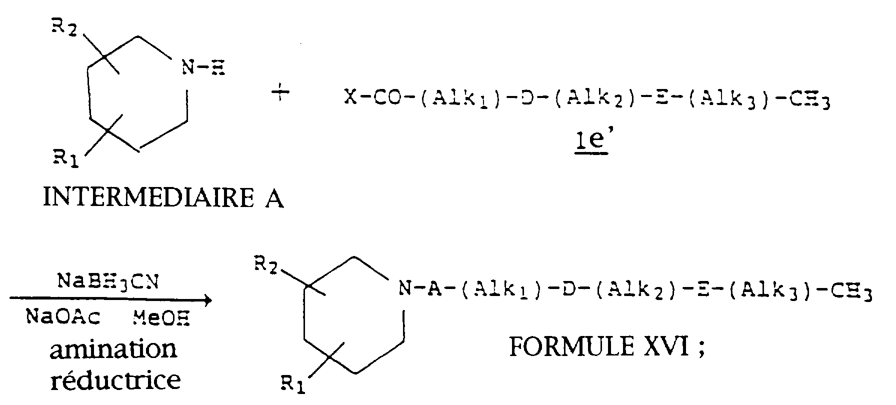 SCHEMA DE REACTION XVII
dans lequel des composés répondant à la formule XVII sont préparés en réduisant un ester de pipéridine substitué par un groupe trifluorométhylalkyle 4e, la formule XVII étant une représentation de la formule A dans laquelle A représente -CH(CF₃)- et R₁, R₂, R', R", R"', D, E, (Alk₁), (Alk₂) et (Alk₃) sont tous définis comme ci-dessus dans la formule A :
